# EUROPEAN PATENT SPECIFICATION

(11) **EP 3 207 149 B1**
(45) Date of publication and mention of the grant of the patent: **07.06.2023**
(21) Application number: 15850568.5
(22) Date of filing: 15.10.2015
(51) Int. Cl.: C12P 13/04, A61K 9/00, A61K 38/00, A61K 47/34, A61K 47/59, A61P 15/08, A61P 35/00

(54) **PHARMACEUTICAL COMPOSITION WITH IMPROVED STABILITY**
PHARMAZEUTISCHE ZUSAMMENSETZUNG MIT VERBESSERTER STABILITÄT
COMPOSITION PHARMACEUTIQUE PRÉSENTANT UNE STABILITÉ AMÉLIORÉE

(30) Priority: 15.10.2014 US 201462064008 P
(43) Date of publication of application: 23.08.2017
(73) Proprietor: Foresee Pharmaceuticals Co., Ltd., Taipei City Taiwan 115 (TW); Li, Yuhua, Landenberg, PA 19350 (US); Guarino, Andrew J., Newark, DE 19702 (US)
(72) Inventor: LI, Yuhua, Landenberg, PA 19350 (US); GUARINO, Andrew, J., Newark, DE 19702 (US)
(74) Representative: Gulde & Partner
(86) International application number: PCT/US2015/055634
(87) International publication number: WO 2016/061296

(56) References cited:
- US-A1- 2003 133 964
- US-A1- 2005 042 294
- US-A1- 2007 196 416
- US-A1- 2008 020 016
- BURTON K W ET AL: "Extended release peptide delivery systems through the use of PLGA microsphere combinations", JOURNAL OF BIOMATERIALS SCIENCE. POLYMER EDIT, VSP, UTRECHT, NL, vol. 11, no. 7, 1 January 2000 (2000-01-01), pages 715-729, XP009079300, ISSN: 0920-5063, DOI: 10.1163/156856200743977
- JAY A. SCHRIER ET AL: "Recombinant Human Bone Morphogenetic Protein-2 Binding and Incorporation in PLGA Microsphere Delivery Systems", PHARMACEUTICAL DEVELOPMENT AND TECHNOLOGY, vol. 4, no. 4, 20 January 1999 (1999-01-20), pages 611-621, XP055472724, US ISSN: 1083-7450, DOI: 10.1081/PDT-100101400

## Description

### FIELD OF THE INVENTION

The field of the invention relates to a delivery system for the sustained and controlled release delivery of bioactive substances. More particularly, the invention relates to a composition of a delivery system for the sustained release delivery of a bioactive substance by means of a biodegradable polymer, and the process for making such a composition.

### BACKGROUND OF THE INVENTION

Biocompatible and biodegradable polymers have been increasingly used as drug delivery carriers to provide sustained or delayed release of bioactive substances. The delivery systems are available in various injectable depot forms including liquid forms, suspensions, solid implants, microspheres, microcapsules and microparticles. Exemplary embodiments of peptide delivery systems using poly(lactide-co-glycolide) (PLGA) are disclosed in Burton et al., "Extended release peptide delivery systems through the use of PLGA microsphere combinations", Journal of Biomaterials Science, 2000, vol. 11, pages 715-729.

Sustained release delivery systems using biocompatible and biodegradable polymers are particularly beneficial for highly potent drugs with a short half-life. Such delivery systems could reduce the frequency of administration and pain, enhance the patient compliance, improve patient convenience, and lower the cost. For many peptide substances, particularly hormones, it requires the drug to be delivered continuously at a controlled rate over a long period of time, and thus a controlled release delivery system is desirable. Such systems may be provided by incorporating the bioactive substances in biodegradable and biocompatible polymer matrices. In one approach, the polymer is dissolved in an organic solvent and then mixed with the bioactive substance that is fabricated into the forms of microparticles, microspheres, microcapsules, microgranules or solid implants by removing the organic solvent. The bioactive substance is entrapped within the solid polymer matrices. Several products have been successfully developed by using biodegradable polymers in the forms of microparticles and solid implants, such as Lupron Depot, Zoladex, Trelstar, Sandostatin LAR, etc. Although these products appear to be effective, they have drawbacks and limitations, such as the large volume of suspending fluids for microparticles, or surgical insertion for solid implants. These products are not very patient friendly. In addition, the manufacturing processes for producing sterile products reproducibly are complicated, resulting in high cost of manufacturing. It is highly desirable that a composition can be easily manufactured and used.

In another approach, the biodegradable polymer and bioactive substances are dissolved in a biocompatible organic solvent to provide a liquid or flowable composition. When the liquid composition is injected into the body, the solvent dissipates into the surrounding aqueous environment, and the polymer forms a solid or gel depot from which the bioactive substance is released over a long period of time. The following references U.S. Pat. Nos. 8,173,148; 8,313,763; 6,565,874; 6,528,080; RE37,950; 6,461,631; 6,395,293; 6,355,657; 6,261,583; 6,143,314; 5,990,194; 5,945,115; 5,792,469; 5,780,044; 5,759,563; 5,744,153; 5,739,176; 5,736,152; 5,733,950; 5,702,716; 5,681,873; 5,599,552; 5,487,897; 5,340,849; 5,324,519; 5,278,202; 5,278,201; and 4,938,763 are believed to be representative in this area. Notwithstanding some success, those methods are not entirely satisfactory for a large number of bioactive substances that would be effectively delivered by such an approach.

Polyester is one of the most popular polymers used in biodegradable sustained drug delivery systems thus far. The polyester and its close relatives, the polyanhydride and polycarbonate, are well-known and have been used in pharmaceutical application for many years. For example, poly(lactide-co-glycolide) or polylactide is the polymeric material used in Lupron Depot and Eligard products for the treatment of advanced prostate cancer. These polyesters are biocompatible and degraded by typical biochemical pathways, such as hydrolysis and enzymolysis, to result in naturally occurring metabolic products. The biodegradability of polyesters is beneficial for use as sustained release drug delivery carriers, but the susceptibility also presents a problem.

Many bioactive substances often contain one or more nucleophilic groups, such as amine groups that can lead to an interaction between the bioactive substance and the biodegradable polymer of the composition. When the bioactive substances and biodegradable polymer are combined, the reaction between nucleophilic groups of the bioactive substances and ester bonds of the polymer can occur. Such a reaction can adversely affect the physical and/or chemical characteristics of the composition resulting in a loss of the advantages of a sustained and controlled delivery system. Many efforts have been taken to address this problem by using acid additives, stabilizing associates, etc. [See US Patents 8,173,148 and 8,343,513].

In addition to the degradation of the polymers, another aspect is the stability of the bioactive substances in the drug delivery system, which is also of critical importance. A significant amount of bioactive substances related impurities could be generated during the fabrication process of the dosage forms, storage, and *in vivo* release. For example, as disclosed in US Patent 6,565,874, example 6, poly(DL-lactide-co-glycolide) with a molar ratio of lactide to glycolide of 75/25 (Birmingham Polymer, Inc.) was dissolved in NMP to give a solution with 45% polymer by weight. This solution was combined and mixed with leuprolide acetate to result in a flowable and injectable viscous formulation. As shown in the present application, a significant amount leuprolide related substances or impurities were unexpectedly observed from this type of formulation over a short period of time which would adversely compromise the quality of the drug product. More surprisingly, the major impurities generated were not the reaction between bioactive substances and the poly(DL-lactide-co-glycolide) as disclosed in the prior art, but the direct reaction between bioactive substances and the residual or unreacted lactide monomers of the polymer.

According to ICH guidelines [http://www.fda.gov/downloads/Drugs/GuidanceComplianceRegulatoryInformation/G uidances/ucm073389.pdf], any impurity (individual impurity) in a new drug product greater than 0.1% has to be reported. Based on maximum daily dose, any impurity greater than 0.1%, 0.2%, 0.5%, or 1% has to be identified. If the impurity in a new drug product is more than a given qualification threshold level, those impurities should be identified and adequately tested for their adverse effects and biological safety. Therefore, any impurity generation exceeding the corresponding qualification threshold will raise regulatory compliance issues. Characterization and qualification of these impurities for their adverse effects and biological safety can be very expensive and time-consuming.

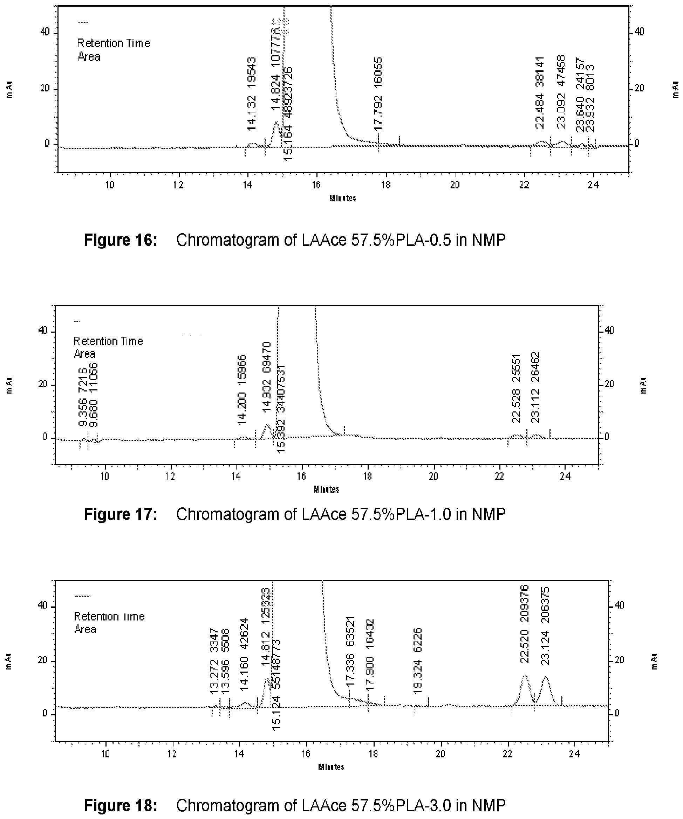

US Patent 8,343,513 disclosed several ways to eliminate or reduce impurities in microspheres. It describes that "the following general considerations should be kept in mind in any efforts to eliminate or reduce impurities in microspheres: (i) Higher the lactide content in PLGA microsphere, lower will be the amount of related substances and the microspheres prepared from 100% PLA will have least amount of related substances; (ii) higher the PLGA molecular weight, higher will be the related substances; higher the target load in PLGA, higher will be the level of the related substances; and (iii) lower the level of extractable oligomers in PLGA, higher will be the level of related substances; hydrophobic PLGA (end blocked PLGA) can produce more related substances compared to the hydrophilic PLGA (free acid end group)" [See US Patent 8,343,513, Column 11, second paragraph]. The overall teaching is to use low molecular weight polyesters having acid end groups with added significant additional amount of low pKa acid additives or oligomers. Examples of acid additives include lactic acid and glycolic acid which are monomer building blocks for the PLGA. The excess amount of acid additives has some limited success to reduce the generation of impurities within a short period of time (24 hours) in non-pharmaceutically acceptable solvents, such as dichloromethane and methanol. In addition, acidic additives cause low pH in the dispersed phase. It is well-known that low pH would cause tissue irritations. Thus, such dispersed phases may be used for manufacturing of microspheres, but are not suitable for administration to patients via direct injection. Additionally, the patent 8,343,513 identifies that the impurities observed in the microspheres containing leuprolide acetate and PLGA50:50 are adducts of the arginine group of leuprolide with the fragments of PLGA [See US Patent 8,343,513, Figure 16 and columns 43 & 44]. These microparticles were prepared using solutions of polymer in non-pharmaceutically acceptable solvents, such as dichloromethane and methanol. The impurities do not represent the entire impurities generated in the solutions. Some of the impurities could be extracted into the aqueous phase during the microparticle preparation processes and could not be detected in the microspheres. Furthermore, the impurities in the microspheres identified are the reaction products between leuprolide and polymer, not the lactide monomers [Murty SB, Thanoo BC, Wei Q, DeLuca PP. Int J Pharm. 2005 Jun 13;297(1-2):50-61. Impurity formation studies with peptide-loaded polymeric microspheres Part I. In vivo evaluation]. Surprisingly, the major impurities generated and described in the present invention were not identified in US Patent 8,343,513 and other prior art.

US Patent 8,951,973 disclosed a way to modulate the release and increase the stability of peptides in microspheres. It describes changing the isoelectric point of the peptide through changing the overall charge on the peptide, which can reduce the burst of the peptide from microspheres and improve the stability. However, this is done by changing an amino acid in the peptide sequence, which makes a new chemical entity. This new chemical entity will require additional work to determine if the same efficacy and safety can be achieved.

Therefore, there is a need to develop controlled release compositions that will minimize or prevent the generation of bioactive substance related impurities and undesirable premature degradation of the biodegradable polymer, and can be injected to patients directly to form a sustained release depot *in situ.*

### SUMMARY OF THE INVENTION

The present invention is directed to subject matter as defined in the claims.

It was unexpectedly discovered that a significant level of impurities are generated rather quickly, in an injectable biodegradable polymeric formulation with a nucleophilic bioactive substance in an organic solvent, even when the acid number of the polymer is larger than 5 mgKOH/g. These impurities are formed through reaction of the nucleophilic bioactive substance with the unreacted or residual monomers of the biodegradable polymer. In solution, the nucleophilic bioactive substance and the polymer/monomer come into intimate contact, creating favorable conditions for reaction to generate impurities/conjugates depending on the choice of solvents.

The present invention shows that polymer compositions can be obtained that have improved stability over the prior art. The conjugates formed in the compositions of the prior art can be substantially reduced or prevented. The present invention provides a stable, injectable, biodegradable polymeric composition for a sustained release delivery system for a nucleophilic bioactive substance and the process for making such polymeric compositions.

The compositions in accordance with the present invention comprise a) a nucleophilic bioactive substance; b) a pharmaceutically acceptable solvent; and c) a suitable biodegradable polymer, that, when formulated together, reduce or prevent the formation of impurities or related substances. The pharmaceutical composition can be a viscous or non-viscous liquid, gel, or cream, which can be injected using a syringe. The pharmaceutical composition is more stable and can be pre-filled into a single syringe, providing a ready to use system.

The bioactive substances of the present invention contain a nucleophilic group that is capable of catalyzing ester degradation and reacting with a lactate-based polymer, oligomer, or monomer. The bioactive substances can be in the form of a peptide, prodrug, or salt thereof. The impurities generated in the composition are adducts between the bioactive substance and the building blocks of the lactate-based polymer (e.g., lactide monomers and oligomers).

According to the present invention, the pharmaceutically acceptable organic solvent may be selected from a group consisting of N-methyl-2-pyrrolidone

(NMP), 2-pyrrolidone, methoxypolyethylene glycol, alkoxypolyethylene glycol, polyethylene glycol esters, glycofurol, glycerol formal, methyl acetate, ethyl acetate, methyl ethyl ketone, dimethylformamide (DMF), dimethylsulfoxide (DMSO), dimethylacetamide (DMAC), tetrahydrofuran (THF), caprolactam, decylmethylsulfoxide, benzyl alcohol, benzyl benzoate, ethyl benzoate, triacetin, diacetin, tributyrin, triethyl citrate, tributyl citrate, acetyl triethyl citrate, acetyl tributyl citrate, triethylglycerides, triethyl phosphate, diethyl phthalate, diethyl tartrate, ethyl lactate, propylene carbonate, ethylene carbonate, butyrolactone, and 1-dodecylazacyclo-heptan-2-one, and combinations thereof.

According to the present invention the biodegradable polymer may be a linear polymer, or a branched polymer, or a mixture of the two. Preferably, the polymer is a lactate-based polymer. The lactate-based polymer includes homopolymers of lactic acid or lactide monomers (poly(lactic acid) or polylactide, PLA), and copolymers of lactic acid (or lactide) with other monomers (for example, glycolic acid, glycolide (poly(lactide-co-glycolide), PLG or PLGA) and the like). The weight average molecular weight of the polymer is typically 5,000 to 50,000. The polymer would ideally have an acid number of less than 3 mgKOH/g, preferably less than 2 mgKOH/g, and more preferably less than 1 mgKOH/g.

According to the present invention, the lactate-based biodegradable polymer can be dissolved in a solvent. The polymer can then be precipitated into an anti-solvent in which the lactate-based polymer is not soluble, but the monomers and oligomers are soluble. The resulting precipitated polymer would ideally have a content of unreacted or residual lactide monomer of 0.3%, preferably 0.2%, and more preferably 0.1% or less. The fraction of oligomers having a molecular weight of less than 5000 would be 20% by weight, preferably 10%, more preferably 5% or less. This polymer, when formulated with the nucleophilic bioactive substance and the pharmaceutically acceptable organic solvent would form a stable solution, which can be prefilled into a single syringe.

According to the present invention, an injectable composition for controlled release drug delivery can be produced by a process comprising: combining a lactate-based polymer having a weight average molecular weight between 5,000 and 50,000 dalton, an acid number of less than 3 mgKOH/g, and a residual lactide monomer in the lactate-based polymer of less than about 0.3% by weight; with a pharmaceutically acceptable organic solvent; and a bioactive substance or a salt thereof capable of reacting with lactide monomer to form a conjugate, with the proviso that no acid additive is added in making the composition.

### Brief Description of the Figures

FIG. 1. Chromatogram of leuprolide acetate in 60%PLA-100DL2E in NMP solution after one hour at 37°C
FIG. 2. Chromatogram of LAAce 60% PLA-100DL2E in NMP at time 0
FIG. 3. Chromatogram of LAAce 60% PLA-100DL2E in DCM at time 0
FIG. 4. Chromatogram of LAAce 60% PLA-100DL2E in DMSO at time 0
FIG. 5. Chromatogram of LAAce 60% PLA-100DL2E in NMP after 1 hour at 37°C
FIG. 6. Chromatogram of LAAce 60% PLA-100DL2E in DMSO after 1 hour at 37°C
FIG. 7. Chromatogram of LAAce 60% PLA-100DL2E in DCM after 1 hour at 37°C
FIG. 8. Chromatogram of FMOC-SER-OH in NMP with 25% D,L-lactide after 3 hours at 25°C
FIG. 9. Chromatogram of FMOC-SER-OH in NMP with 25% D,L-lactide after 1 day at 25°C
FIG. 10. Chromatogram of FMOC-ARG-OH in NMP with 25% D,L-lactide after 3 hours at 25°C
FIG. 11. Chromatogram of FMOC-ARG-OH in NMP with 25% D,L-lactide after 1 day at 25°C
FIG. 12. Chromatogram of LAMS in NMP with 10% L-lactide showing impurities generated from monomers
FIG. 13. Chromatogram of LAAce 57.5%PLA-0.1 in NMP
FIG. 14. Chromatogram of LAAce 57.5%PLA-0.2 in NMP
FIG. 15. Chromatogram of LAAce 57.5%PLA-0.3 in NMP
FIG. 16. Chromatogram of LAAce 57.5%PLA-0.5 in NMP
FIG. 17. Chromatogram of LAAce 57.5%PLA-1.0 in NMP
FIG. 18. Chromatogram of LAAce 57.5%PLA-3.0 in NMP
FIG. 19. Chromatogram of LAAce 57.5%PLA-0.1 in NMP after 24 hr at 37°C
FIG. 20. Chromatogram of LAAce 57.5%PLA-0.2 in NMP after 24 hr at 37°C
FIG. 21. Chromatogram of LAAce 57.5%PLA-0.3 in NMP after 24 hr at 37°C
FIG. 22. Chromatogram of LAAce 57.5%PLA-0.5 in NMP after 24 hr at 37°C
FIG. 23. Chromatogram of LAAce 57.5%PLA-1.0 in NMP after 24 hr at 37°C
FIG. 24. Chromatogram of LAAce 57.5%PLA-3.0 in NMP after 24 hr at 37°C
FIG. 25. *In vitro* release of LAMS from formulations with purified or unpurified polymer

### DETAILED DESCRIPTION OF THE INVENTION

The present invention provides a polymeric composition for forming a sustained release delivery system for bioactive substances and the process of making such composition. The polymeric compositions of the present invention comprise a) a bioactive substance or salt thereof; b) an organic solvent; and c) a lactate-based biodegradable homopolymer or copolymer. The bioactive substances or their salts thereof of the present invention are typically nucleophilic and can react with lactide monomers or lactate-based oligomers to form covalent conjugates or adducts. The organic solvents of the present invention can be a polar protic or an aprotic liquid. The lactate-based polymers of the present invention contain at least one monomer unit of lactic acid, lactate, or lactide in the polymer composition structure. The polymeric compositions of the present invention are capable of reducing or preventing the reaction of bioactive substance with monomer or oligomer to form related impurities in the polymer compositions.

The polymeric composition of the present invention may be in the forms of microparticles, microspheres, microcapsules, microgranules or solid implants by removing the organic solvent prepared *in vitro.* These dosage forms can be administered by methods known in the art, such as by injection or surgical intervention. Alternatively and preferably, it may be in the forms of solutions, emulsions, suspensions, paste, cream, or gel which moves as a fluid so that it may be injected through a needle, cannula, tube, laproscope, probe, or other delivery device. When administered to a subject, such injectable composition forms a depot *in-situ* from which the controlled release of the bioactive substance can be sustained for a desired period of time depending upon the composition. The depot or implant may be a solid, a gel, a paste, a semisolid, or a viscous liquid. With the selections of the biodegradable polymer and other components, the duration of the sustained release of the bioactive substance can be controlled over a period of time from several weeks to one year.

The polymeric composition of the present invention may also include non-polymeric compounds, and/or additives for controlling release, such as rate release modulating agents, pore forming agents, plasticizers, organic solvents, encapsulation agents for encapsulating the bioactive substance, thermal gelling agents, burst effect reducing materials, hydrogels, polyhydroxyl materials, leaching agents, tissue transporting agents, or other similar additives or any combination thereof.

The terms "a", "an" and "one", as used herein, are meant to be interpreted as "one or more" and "at least one."

The term "controlled release delivery", as defined herein, is intended to refer to the delivery of a bioactive substance *in vivo* over a desired, extended period of time following administration, preferably from at least a few days to one year.

The term "bioactive substance" is meant to include any materials having diagnostic and/or therapeutic properties including, but not limited to, organic small molecules, inorganic small molecules, macromolecules, peptides, oligopeptides, proteins, or enzymes, nucleotides, nucleosides, oligonucleotides, oligonucleosides, polynucleotides, polynucleotides, polynucleic acids or similar molecules constitute such chemical compounds. Non-limiting examples of therapeutic properties are antimetabolic, antifungal, anti-inflammatory, antitumoral, antiinfectious, antibiotics, nutrient, agonist, and antagonist properties.

The bioactive substances of the present invention may be in the form of a free molecule, an organic or inorganic salt of the free molecule, or it may be complexed or covalently conjugated with a carrier agent, may be a pro-drug, or may be a multiform bioactive substance (multiple units of the bioactive substance either complexed or covalently bonded together).

The bioactive substances of the present invention contain a nucleophilic group that is capable of catalyzing ester degradation and reacting with lactate-based polymer, oligomer, or monomer. A "nucleophilic group" can be characterized as a chemical species that donates an electron pair to an electrophile to form a chemical bond in relation to a reaction that seeks the nucleus of an atom or the positive end of a polar molecule. All molecules or ions with a free pair of electrons or at least one pi bond are nucleophilic groups. Because nucleophilic groups donate electrons, they are by definition Lewis bases. The nucleophilic groups include nitrogen groups such as an amine group, an amidine group, an imine group, a nitrogen-heteroaromatic group, a nitrogen-heterocyclic group, any other nitrogen containing group or any combination thereof as the nucleophilic group or groups. The nucleophilic nitrogen group or groups may be basic as in the free molecule or may be in salt form with an organic or inorganic acid. The nucleophilic groups may also include oxygen groups such as hydroxide anion, alcohols, alkoxide anions, hydrogen peroxide, and carboxylate anions and sulfur groups such as hydrogen sulfide and its salts, thiols (RSH), thiolate anions (RS-), anions of thiolcarboxylic acids (RC(O)-S-), and anions of dithiocarbonates (RO-C(S)-S-) and dithiocarbamates (R2N-C(S)-S-).

The bioactive substance of the present invention may be an aliphatic, aromatic, heteroaromatic, cyclic, alicyclic, heterocyclic organic compound optionally containing one or more carboxylic acid, ester, lactone, anhydride, carbonate, carbamate, urea, amide, lactam, imine, amidine, enamine, imide, oxime, carbonyl, hydroxyl, enol, amine, ether, sulfide, sulfonyl, sulfoxyl, sulfonic acid, thioamide, thiol, thioacid, thioester, thiourea, acetal, ketal, halide, epoxy, nitro, nitroso, xanthate, ynamine group or any combination thereof wherein the optional substituents are compatible with the nucleophilic group of the bioactive substance.

The term "peptide" as used herein is in a generic sense to include poly(amino acids) that are normally generally referred to as "peptides", "oligopeptides", and "polypeptides" or "proteins" which are used interchangeably herein. The term also includes bioactive peptide analogs, derivatives, acylated derivatives, glycosylated derivatives, pegylated derivatives, fusion proteins and the like. The term "peptide" is meant to include any bioactive peptides having diagnostic and/or therapeutic properties including, but not limited to, antimetabolic, antifungal, anti-inflammatory, antitumoral, antiinfectious, antibiotics, nutrient, agonist, and antagonist properties. The term also includes synthetic analogues of peptides, unnatural amino acids having basic functionality, or any other form of introduced basicity. The peptide of the present invention contains at least one nucleophilic group. The phrase "at least one" means that the peptide may also contain a multiple number of nucleophilic groups.

Specifically, the bioactive peptides of the invention are luteinizing hormone releasing hormone (LHRH), LHRH agonists, LHRH antagonists and synthetic analogues and modifications and pharmacologically-active fragments thereof.

The preferred peptides used herein contains an amino acid serine in the peptide molecular structure. The preferred peptides used herein include LHRH, and LHRH agonists such as leuprorelin, buserelin, gonadorelin, deslorelin, fertirelin, histrelin, lutrelin, goserelin, nafarelin, triptorelin, cetrorelix, enfuvirtide, thymosin α1, abarelix. The preferred peptide used herein also includes peptides such as somatostatin, octreotide, pasireotide, SOM230, and lanreotide.

The bioactive substances of the present invention also include nucleotides, nucleosides, oligonucleotides, oligonucleoside and polynucleic acids that are biologically active compounds having nucleophilic capabilities.

The bioactive substance used in the present invention may be itself or a pharmaceutically acceptable salt. The acid used to form the pharmaceutically acceptable salt of the bioactive substance preferably has a pKa less than 5. The acids suitable for the present invention may be selected from, but not limited to, the group consisting of hydrochloric acid, hydrobromic acid, nitric acid, chromic acid, sulfuric acid, methanesulfonic acid, trifluromethane sulfonic acid, trichloroacetic acid, dichloroacetic acid, bromoacetic acid, chloroacetic acid, cyanoacetic acid, 2-chloropropanoic acid, 2-oxobutanoic acid, 2-chlorobutanoic acid, 4-cyanobutanoic acid, pamoic acid, perchloric acid, phosphoric acid, hydrogen iodide, acetic acid, 2,2-dichloroacetic acid, adipic acid, alginic acid, L-ascorbic acid, L-aspartic acid, benzenesulfonic acid, benzoic acid, 4-acetamido benzoic acid, (+)-camphoric acid, (+)-camphor-10-sulfonic acid, capric acid, (decanoic acid), caproic acid (hexanoic acid), caprilic acid (octanoic acid)carbonic acid, cinnamic acid, citric acid, cyclamic acid, decanoic acid, dodecylsulfuric acid, ethane-1,2-disufonic acid, ethanesulfonic acid, 2-hydroxy-ethanesulfonic acid, formic acid, fumaric acid, galactic acid, gentisic acid, D-glucoheptonic acid, D-gluconic acid, D-glucuronic acid, glutamic acid, glutaric acid, 2-oxo-glutaric acid, glycerophosphoric acid, glycolic acid, hippuric acid, isobutyric acid, DL-lactic acid, lactobionic acid, lauric acid, maleic acid, (-)-L-malic acid, malonic acid, DL-mandelic acid, muric acid, naphthalene-1,5-disulfonic acid, naphthalene-2-sulfonic acid, 1-hydroxy-2-naphthoic acid, nicotinic acid, oleic acid, orotic acid, oxalic acid, palmitic acid, embonic acid, proprionic acid, (-)-L-pyroglutamic acid, salicyclic acid, 4-amino-salicylic acid, sebacic acid, stearic acid, succinic acid, (+)-L-tartaric acid, thiocyanic acid, p-toluenesulfonic acid, undecylenic acid. The selection of the suitable acids is well-known to those of skill in the art.

The pharmaceutically acceptable salt of the bioactive substance can be prepared by simple acid and base titration or neutralization. The pharmaceutically acceptable salt of the bioactive substance can be prepared during its synthesis and purification processes. Alternatively, the salts can be prepared from bioactive substance in the form of a free base. The free base is dissolved in a suitable liquid medium. This solution of the bioactive substance is mixed with a solution of an acid to form the beneficial salts by removing the solvent through suitable means, such as filtration, precipitation, or lyophilization. If the bioactive substance is in its common commercially available salt form, a different salt can be obtain by using a simple salt exchange process or ion-exchange method such as lyophilization, precipitation or other methods known in the art. For example, leuprolide acetate is dissolved in a suitable liquid medium, e.g., water. This solution of the peptide is mixed with an aqueous solution of a strong acid, such as methanesulfonic acid. When the leuprolide acetate and a strong acid, such as methanesulfonic acid are dissolved in water, the peptide tends to be associated with mesylate ion, as the stronger methanesulfonic acid displaces the weaker carboxylic acetic acid. The solvent and liberated acetic acid (or other weak but volatile carboxylic acid) may be removed under vacuum. Thus, the mixture solution is freeze-dried to remove water and weaker acid to form the desired salts. If the bioactive substance is not stable under low pH, the pharmaceutically acceptable salts of the bioactive substance can be prepared through extensive dialysis against very low concentration of an acid.

The polymer compositions of the present invention may contain bioactive substance in a range of 0.01 to 40% by weight. In general, the optimal drug loading depends upon the period of release desired and the potency of the bioactive substance. Obviously, for bioactive substance of low potency and longer period of release, higher levels of incorporation may be required.

The term "organic solvent" is meant to include any organic solvents that can dissolve the lactate-based polymers. Typical solvents that may be used in the polymeric composition of the present invention include water, methanol, ethanol, dimethyl sulfoxide (DMSO), dimethyl formamide, dimethyl acetamide, dioxane, tetrahydrofuran (THF), acetonitrile, methylene chloride, ethylene chloride, carbon tetrachloride, chloroform, lower alkyl ethers such as diethyl ether and methyl ethyl ether, hexane, cyclohexane, benzene, acetone, ethyl acetate, and the like. Esters of carbonic acid and aryl alcohols such as benzyl benzoate; C4 to C10 alkyl alcohols; C1 to C6 alkyl C2 to C6 alkanoates; esters of carbonic acid and alkyl alcohols such as propylene carbonate, ethylene carbonate and dimethyl carbonate, alkyl esters of mono-, di-, and tricarboxylic acids, such as 2-ethoxyethyl acetate, ethyl acetate, methyl acetate, ethyl butyrate, diethyl malonate, diethyl glutonate, tributyl citrate, diethyl succinate, tributyrin, isopropyl myristate, dimethyl adipate, dimethyl succinate, dimethyl oxalate, dimethyl citrate, triethyl citrate, acetyl tributyl citrate, glyceryl triacetate; alkyl ketones such as methyl ethyl ketone; as well as other carbonyl, ether, carboxylic ester, amide and hydroxy containing liquid organic compounds having some solubility in water. Propylene carbonate, ethyl acetate, triethyl citrate, isopropyl myristate, and glyceryl triacetate are preferred because of biocompatibility and pharmaceutical acceptance. Selection of suitable solvents for a given system will be within the skill in the art in view of the present disclosure.

Preferably, the organic solvents of the present invention are biocompatible and pharmaceutically acceptable. The term "biocompatible" means that the organic solvent as it disperses or diffuses from the composition does not result in substantial tissue irritation or necrosis surrounding the implant site. The term "pharmaceutically acceptable" means that the organic solvents can be used in a drug product to treat humans and animals in need.

The organic solvents of the present invention may be miscible or dispersible in aqueous or body fluid. The term "dispersible" means that the solvent partially soluble or miscible in water. A single solvent or a mixture of solvents may have a solubility or miscibility in water of greater than 0.1% by weight. Preferably, the solvent has a solubility or miscibility in water of greater than 3% by weight. More preferably, the solvent has a solubility or miscibility in water of greater than 7% by weight. The suitable organic solvent should be able to diffuse into body fluid so that the liquid composition coagulates or solidifies. Single and/or mixture of such solvents can be employed; the suitability of such solvents can be determined readily by simple experimentations.

Examples of pharmaceutically acceptable organic solvent include, but not limited to, N-methyl-2-pyrrolidone (NMP), 2-pyrrolidone, methoxypolyethylene glycol, alkoxypolyethylene glycol, polyethylene glycol esters, glycofurol, glycerol formal, methyl acetate, ethyl acetate, methyl ethyl ketone, dimethylformamide (DMF), dimethylsulfoxide (DMSO), dimethylacetamide (DMAC), tetrahydrofuran (THF), caprolactam, decylmethylsulfoxide, benzyl alcohol, benzyl benzoate, ethyl benzoate, triacetin, diacetin, tributyrin, triethyl citrate, tributyl citrate, acetyl triethyl citrate, acetyl tributyl citrate, triethylglycerides, triethyl phosphate, diethyl phthalate, diethyl tartrate, ethyl lactate, propylene carbonate, ethylene carbonate, butyrolactone, and 1-dodecylazacyclo-heptan-2-one, and combinations thereof. Preferred organic solvents include N-methyl-2-pyrrolidone, 2-pyrrolidone, dimethylsulfoxide, dimethylacetamide (DMAC), ethyl lactate, glycofurol, glycerol formal, benzyl alcohol, benzyl benzoate, methoxypolyethylene glycol, alkoxypolyethylene glycol, polyethylene glycol esters, and isopropylidene glycol.

The solubility of the biodegradable polymers in various organic solvents will differ depending upon the characteristics of the polymers and their compatibility with the solvents. Thus, the same polymer will not be soluble to the same extent in different solvents. For example, PLGA has a much higher solubility in N-methyl-2-pyrrolidone (NMP) than that in triacetin. However, when PLGA solution in NMP is in contact with aqueous solution, NMP will dissipate very rapidly to form a solid polymer matrix due to its high water miscibility. The fast diffusion rate of the solvent may result in a solid implant forming quickly, however, it may also lead to a high initial burst release. When PLGA solution in triacetin is in contact with aqueous solution, triacetin will dissipate very slowly due to its low water miscibility. The slow diffusion rate of the solvent may take a long time to transform from a viscous liquid to a solid matrix. There may be an optimum balance at which the solvent diffuse out and the coagulation of the polymer to encapsulate peptide substances. Therefore, it may be advantageous to combine different solvents to obtain a desirable delivery system. The solvents of low and high water miscibility may be combined to improve the solubility of the polymer, modify the viscosity of the composition, optimize the diffusion rate, and reduce the initial burst release.

The polymeric compositions of the present invention typically contain an organic solvent in a range of 10% to 99% by weight. The viscosity of the polymeric compositions of the invention depends on the molecular weight of the polymer and organic solvent used. Preferably the concentration of the polymer in the compositions is less than 70% by weight.

A "polymer" is a large molecule, or macromolecule, composed of many repeated subunits. Polymers range from familiar synthetic plastics such as polystyrene to natural biopolymers such as DNA and proteins that are fundamental to biological structure and function. Polymers, both natural and synthetic, are created via polymerization of many small molecules, known as monomers. Polymerization is the process of combining many small molecules known as monomers into a covalently bonded chain or network. The polymer large molecular mass relative to small molecule compounds produces unique physical properties, including toughness, viscoelasticity, and a tendency to form glasses and semicrystalline structures rather than crystals.

The term "biodegradable" refers to a material that gradually decomposes, dissolves, hydrolyzes and/or erodes *in situ.* Generally, the "biodegradable polymers" herein are polymers that are hydrolyzable, and/or bioerode *in situ* primarily through hydrolysis and/or enzymolysis.

The term "biodegradable polymer" as used herein is meant to include any biocompatible and/or biodegradable synthetic and natural polymers that can be used *in vivo.* Generally, the biodegradable polymer of the present invention may be a linear polymer, or a branched or star polymer, or a mixture of a linear polymer and a branched and/or star polymer. Preferably, the biodegradable polymer of the present invention is lactate-based polymer. The "lactate-based polymer" as used herein is a polymer that contains a lactate unit in the polymer. The term "lactate" as used herein refers to either the lactic acid, or its salts (lactates) which are used as reagents in preparation of lactate-based polymers, or refer to those moieties as residues incorporated via ester bonds into the lactate-based polymer molecular chains. The term "lactate" as used herein also refers to the cyclic dimeric ester of lactate (lactide) when referring to monomer used in preparation of lactate-based polymers. The lactide monomer is a natural and renewable compound produced from lactic acid (2-hydroxypropanoic acid). Lactide, as a product of lactic acid, which has two stereoisomeric forms (L(+)lactic acid and D(-)lactic acid), exists in three stereoisomeric forms: L-lactide, D-lactide and Meso-lactide.

Lactide is obtained in two synthesis steps: oligomerization of lactic acid followed by cyclization. L-lactide is produced if the original acid is L-lactic acid and D-lactide is produced if the original acid is D-lactic acid. Meso-lactide is produced by using combination of L-lactic acid and D-lactic acid. An efficient purification step is necessary to obtain the right level of purity for the polymerization of lactide into PLA *(*Savioli Lopes M., Jardini A., Maciel Filho R., 2014, Synthesis and characterizations of poly (lactic acid) by ringopening polymerization for biomedical applications, Chemical Engineering Transactions, 38, 331-336 DOI: 10.3303/CET1438056*].*

It is understood that when the terms "lactic acid," "lactate," or "lactide" are used herein, that any and all chiral forms of the compounds are included within the terms. Thus, "lactic acid" includes (*R*)-lactic acid and (*S*)-lactic acid or D-lactic acid, L-lactic acid, D,L-lactic acid, or any combination thereof; "lactide" includes D-lactide, D,L-lactide, L,D-lactide, L-lactide, (*R*,*R*)-Lactide, (*S*,*S*)-lactide and *meso*-lactide or any combination thereof.

Lactate-based polymers include any polymers/copolymers that contain lactate, lactic acid, or lactide monomers. The lactate-based polymers can be prepared by polycondensation (PC), ring-opening polymerization (ROP), and other methods (chain extension, grafting). The different types of polymers, including copolymers, can be prepared by ROP from D,L-lactide, L-lactide, D-lactide, glycolide (GA), ε-caprolactone (CL), trimethylene carbonate (TMC), 1,5-dioxepan-2-one (DXO), and other cyclic analogues.

The lactate-based polymer of the present invention includes homopolymers of lactic acid or lactide monomers (poly(lactic acid) or polylactide, PLA), and copolymers of lactic acid (or lactide) with other monomers (for example, glycolic acid (or glycolide) (poly(lactide-co-glycolide), PLG or PLGA) and the like). The lactate-based polymer may have the same end groups, i.e., all the end groups are the same, such as ester, or hydroxyl or carboxylic acid. The lactate-based polymer may have mixed end groups of ester, hydroxyl, and/or carboxylic acid. The lactate-based polymer can have a diol core with end hydroxyl groups, such as those examples disclosed in US Patent 8,470,359. Similarly, the lactate-based polymer may have a triol or polyol core, such as glucose, with end hydroxyl groups. The lactate-based polymer may have one end group as an ester and the other end with a hydroxyl group or carboxylic acid group. The lactate-based polymer may also have one end hydroxyl group and the other end with a carboxylic acid or an ester, or vice versa.

The lactate-based polymer of the present invention has a weight-average molecular weight of usually from 5,000 to 50,000. The lactate-based polymer of the present invention may be a commercially available product or a polymer prepared by a known method. The known polymerization methods, for example, include condensation polymerization of lactic acid and copolymerization with other monomers, such as glycolic acid, ring-opening-polymerization of lactide using a catalyst, such as Lewis acids, or metal salts, such as diethylzinc, triethylaluminum, tin octylate, and copolymerization with other cyclic monomers, such as glycolide; ring-opening-polymerization of lactide in the further presence of a hydroxycarboxylic acid derivative of which carboxyl group is protected (for example, International Patent Publication WO00/35990); ring-opening-polymerization of lactide in which a catalyst is added under heat to lactide to cause ring-opening polymerization (for example, J. Med. Chem., 16, 897 (1973)); and other methods for copolymerization of lactide with glycolide and/or other monomers.

The polymerization can be carried out by bulk polymerization in which lactide and other co-monomers are melted, or by solution polymerization in which lactide and other co-monomers are dissolved in a suitable solvent. The solvent for dissolving lactide in solution polymerization includes, but not limited to, aromatic hydrocarbons, such as benzene, toluene, xylene and the like, decalin, dimethylformamide and the like.

Polymer molecular weight is important because it determines many physical properties. Some examples include the temperatures for transitions from liquids to waxes to rubbers to solids, and mechanical properties, such as stiffness, strength, viscoelasticity, toughness, and viscosity. It is important to select an appropriate polymer with suitable molecular weight for a specific application.

The terms "weight-average molecular weight, Mw" and "number-average molecular weight, Mn" are well-known to those of skill in the art (See http://www.chem.agilent.com/Library/technicaloverviews/Public/5990-7890EN.pdf). The term "polydispersity index, PDI" as used herein is defined as the weight-average molecular weight of a polymer divided by the number-average molecular weight of the polymer (PDI = Mw/Mn). The polydispersity index is well-known to characterize the distribution of molecular weights in a polymer. PDI provides an idea about the homogeneity of a polymer. The polymers whose molecules have nearly same molecular weights are called monodispersed polymers. For these molecules, MW = MN and therefore, the PDI is one. The polymers whose molecules have wide range of molecular weights are called polydispersed polymers. For these polymers, MW > MN and therefore, their PDI is greater than one. The higher the PDI, the broader the distribution of molecular weight of the polymer. The PDI of the lactate-based polymer of the present invention should be less than 2.5, preferably less than 2.0, and more preferably less than 1.8.

The lactate-based polymer of the present invention may be subject to reprecipitation. About 10 to 40% by weight of a lactate-based polymer having a weight-average molecular weight of from 5,000 to 50,000 can be added into a solvent capable of dissolving the lactate-based polymer. The solvent, for example, includes chloroform, dichloromethane, toluene, o-xylene, m-xylene, p-xylene, tetrahydrofuran, acetone, acetonitrile, N-methyl-2-pyrrolidone, DMSO, and N,N-dimethylformamide. The organic solution containing the lactate-based polymer of the present invention can then be precipitated into an anti-solvent in which the lactate-based polymer of the present invention is not soluble. The anti-solvent includes, but not limited to, alcohols such as methanol and ethanol, short chain ethers such as ethyl ether, aliphatic hydrocarbons such as hexane, and water. The monomers and small oligomers of the lactate-based polymer are still soluble in the anti-solvent and so stay in the solution and do not precipitate.

The amount of the anti-solvent which can precipitate the lactate-based polymer is typically from 0.1 to 10-fold by weight, preferably from 0.2 to 5-fold by weight based on the solvent of the lactate-based polymer solution. For example, when 20 grams of the lactate-based polymer of the present invention is dissolved in 100g of acetone, then an anti-solvent, such as water, in an amount of 0.1 to 10-fold by weight based on the acetone is combined with the lactate-based polymer solution to precipitate the polymer.

The precipitation procedure can be performed as one of the following methods: 1) a lactate-based polymer solution in an organic solvent is added all at once into an anti-solvent; 2) a lactate-based polymer solution is added drop-wise into an anti-solvent; 3) an anti-solvent is added all at once into a lactate-based polymer solution; 4) an anti-solvent is added drop-wise into a lactate-based polymer solution, and the like.

The lactate-based polymer of the present invention may be purified by employing supercritical fluid extraction (SFE). SFE is the process of separating one component (the extractant) from another (the matrix) using supercritical fluids as the extracting solvent. Extraction is usually from a solid matrix, but can also be from liquids. SPE employs a fluid in a supercritical state, as is defined for the particular fluid composition in terms of pressure and temperature. Every fluid material has a characteristic combination of pressure and temperature termed a "critical point," and once those parameters are exceeded, the fluid exists in the supercritical state. The fluid or solvent employed in supercritical fluid extraction may be a single compound or may be a mixture of compounds. The fluid components are well known and readily available to those of skill in the art to select suitable solvent and co-solvent to purify the lactate-based polymer of the present invention.

The lactate-based polymer of the present invention also includes block copolymers, such as A-B-A block copolymers, B-A-B block copolymers, and/or A-B block copolymers and/or branched copolymers. The preferred block copolymers are those wherein the A block comprises a lactate-based polymer and the B block comprises a polymer selected from polyglycolides, poly(lactide-co-glycolide)s, polyanhydrides, poly(ortho ester)s, polyetheresters, polycaprolactones, polyesteramides, poly(ε-caprolactone)s, poly(hydroxybutyric acid)s, and blends and copolymers thereof. The B block can also be a polyethylene glycol or monofunctionally derivatized polyethylene glycol, such as methoxy polyethylene glycol. Some of these combinations may form acceptable thermal reversible gels.

According to the present invention, a polymeric composition for controlled release drug delivery is a homogeneous solution of a nucleophilic drug and a polymer in a solvent. Impurities or bioactive substance related substances referred to herein are adducts between the bioactive substance and the building blocks of the lactate-based polymer (e.g., lactic acid, lactate, lactide monomer and oligomers). The impurity problem is more common when a homogeneous solution of a nucleophilic bioactive substance and a polymer is used. In solution, the nucleophilic bioactive substance and the polymer together forms a favorable condition for bioactive substance and polymer/oligomer/monomer to interact/react because of the intimate contact between the bioactive substance and the polymer/oligomer/monomer.

The bioactive substance related substances can be detected by HPLC analysis. As disclosed in US Patent 8,343,513 (col 43 and 44, Table 35 & Fig. 16), 4 leuprolide related impurities were detected by HPLC and HPLC-MS in the PLGA (RG503H) microspheres prepared using solvent extraction method. The microspheres were prepared from a dispersed phase consisting of leuprolide acetate, PLGA (RG503H), dichloromethane (DCM) and methanol. Both solvents are toxic and are not suitable for human use. In one embodiment of the present invention it was found that in pharmaceutically acceptable solvents, such as N-methylpyrrolidone (NMP) and dimethylsulfoxide (DMSO), more bioactive substance related impurities are generated than when in toxic solvents, such as DCM.

The 4 leuprolide related impurities detected by HPLC and HPLC-MS in US Patent 8,343,513 were all found to have formed from reaction at the arginine residue of leuprolide with fragments of the polymer. In one embodiment of the present invention, when lactide monomers were mixed with arginine or serine and dissolved in N-methylpyrrolidone (NMP), a pharmaceutically acceptable solvent, surprisingly, quite different impurity profiles by HPLC were observed. Serine was found to be much more reactive than arginine with the lactide monomers. When leuprolide acetate was mixed with PLGA in NMP, two major leuprolide related impurities were detected by HPLC. The leuprolide related substances found by HPLC were analyzed by ESI-MS/MS to obtain their fragment ion profiles. Based on the MS/MS data, a 144 Da addition at ₄Serine was observed. Conclusively, these two impurities contained the same MW and should be modified at ₄Serine compared to the MS fragments of Leuprolide. These two impurities are leuprolide-lactide conjugates formed by the reaction of serine of leuprolide and the lactide monomers. Two major conjugates were [Pyr-His-Trp-(Ser-**D-Lactide**)-Tyr-D-Leu-Leu-Arg-Pro-NHEt] and [Pyr-His-Trp-(Ser-**L-Lactide**)-Tyr-D-Leu-Leu-Arg-Pro-NHEt] (Pyr = L-Pyroglutamyl) and not detected as disclosed in US Patent 8,343,513. This indicates that the presence of lactide monomers is detrimental to the stability of leuprolide.

Further surprisingly, the formation of these impurities and the molecular weight reduction of the polymer were not prevented by using a low molecular weight polymer with high acid number in these polymeric compositions containing a PLA (MW 11k and acid number 12 mgKOH/g). In fact, the leuprolide-lactide conjugates formed faster in the formulation with the higher acid number. Also, when a poly(lactide-co-glycolide) (PLGA 5050) with an acid number of 5 mgKOH/g was used in these polymeric compositions, it was seen that the higher content of the lactide monomer in the solution resulted in more impurity generation. These findings are unexpected from the disclosure in US Patent 8,343,513. In contrast to the teaching of US Patent 8,343,513, the presence of oligomers did not reduce, but increased the generation of the overall impurities.

US Patent 8,343,513 further discloses that in order to reduce the impurities generation, low molecular weight polymers with high acid numbers and significant amount of low pKa acid additives or oligomers have to be used. Such acidic dispersed phases are not suitable for human parenteral use due to tissue irritation from the low pH. In another embodiment of the present invention, when octreotide mesylate with excess methane sulfonic acid, such that the pH of the salt solution is 2.4, is dissolved in a pharmaceutically acceptable solvent with added lactide monomer, there are surprisingly many impurities generated and the peptide is highly unstable. In fact more impurities are generated for the solution with excess acid than the one without.

According to the present invention, it was surprisingly and unexpectedly discovered that the impurities generation can be reduced or prevented by (1) using lactate-based polymers with low content of residual lactide monomers; (2) using lactate-based polymers with low extractable oligomers; (3) using lactate-based polymers with low acid numbers; and (4) avoiding use of any acid additives.

According to the present invention, the lactate-based polymers have a weight-average molecular weight of from 5,000 to 50,000, 5,000 to 45,000, 5,000 to 40,000, 5,000 to 35,000, 5,000 to 30,000, 5,000 to 25,000, 5,000 to 20,000, 5,000 to 15,000, 5,000 to 12,000, or 10,000 to 40,000, or 12,000 to 35,000, or 15,000 to 30,000 Dalton.

The lactate-based polymers of the present invention have a content of residual or unreacted lactide to be less than 0.3%, preferably less than 0.2%, and more preferably less than 0.1%.

The lactate-based polymers of the present invention have a fraction of oligomers having MW less than 5000 to be less than 20% by weight, preferably less than 15%, preferably less than 10%, and most preferably less than 5%. The lactate-based polymers of the present invention have a fraction of oligomers having MW less than 1000 to be less than 5% by weight, preferably less than 3%, more preferably less than 2%, and most preferably less than 1%.

The polydispersity of the lactate-based polymer of the present invention is from 1.1 to 2.5. Preferably, the polydispersity of the lactate-based polymer of the present invention is at least 2.0 or less. More preferably, the polydispersity of the lactate-based polymer of the present invention is at least 1.8 or less.

In addition, "acid number" of the lactate-based polymers is another critical property that can affect the generation of impurities. Acid number of the polymer is the "mg" amount of potassium hydroxide required to neutralize the acid present in one gram of the polymer. Polymers with acid ended groups will have some acid number. Lower molecular weight polymers will have more acid ended groups, and will have higher acid numbers. Extractable oligomer acids in polymers may also contribute to the acid number. Typically, for polymers with acid ended groups, acid number shows a relationship to molecular weight, more towards the number average molecular weight. The acid number of the lactate-based polymers of the present invention is from 0 to 30 mgKOH/g. The lactate-based polymers of the present invention have an acid number to be less than 20, preferably less than 10, more preferably less than 3, and most preferably less than 2.

The pharmaceutical compositions of the present invention may contain a lactate-based polymer in a range of 5% to 75% by weight. The viscosity of the pharmaceutical compositions of the present invention depends on the molecular weight of the polymer and organic solvent used. Typically, when the same solvent is used, the higher the molecular weight and the concentration of the polymer, the higher the viscosity. Preferably the concentration of the polymer in the compositions is less than 70% by weight.

Lactate-based polymers such as poly(lactic acid), and copolymers of lactic acid and glycolic acid (PLGA), including poly(D,L-lactide-co-glycolide) and poly(L-lactide-co-glycolide) are preferably used in the present invention. The thermoplastic polyesters have monomer ratios of lactic acid to glycolic acid of between about 50:50 to about 100:0 and weight average molecular weights of between about 5,000 to about 50,000. The biodegradable thermoplastic polyesters can be prepared using the methods known in the art, e.g., polycondensation and ring-opening polymerization (e.g., U.S.Pat. No. 4,443,340; 5,242,910; 5,310,865). The biodegradable polymers can also be purified to remove residual monomers and oligomers using the methods known in the art, such as dissolving and re-precipitating the polymer (e.g. U.S. Pat No. 4,810,775; 5,585,460). The terminal groups of the poly(DL-lactide-co-glycolide) can either be hydroxyl, carboxylic, or ester depending upon the method of polymerization and end group modification. The suitable polymers may include a monofunctional alcohol or a polyol residue. Examples of monofunctional alcohols are methanol, ethanol, or 1-dodecanol. The polyol may be a diol, triol, tetraol, pentaol and hexaol including ethylene glycol, 1,6-hexanediol, polyethylene glycol, glycerol, saccharides, glucose, sucrose, reduced saccharides such as sorbitol, and the like. Many suitable PLGAs are available commercially, and the PLGAs of specific compositions can be readily prepared according to the prior art.

The type, molecular weight, and amount of biodegradable polymer present in the compositions can influence the length of time in which the bioactive substance is released from the controlled release implant. The selection of the type, molecular weight, and amount of biodegradable polymer present in the compositions to achieve desired properties of the controlled release implant can be determined by simple experimentations.

In one preferred embodiment of the present invention, the polymeric composition can be used to formulate a controlled release delivery system for leuprolide mesylate. In such an embodiment, the lactate-based polymer can preferably be poly (D,L-lactide-co-glycolide) containing 75% lactide in the polymer chain or higher, a hydroxyl terminal group and a lauryl ester terminus; can be present in about 30% to about 65% of the composition by weight; and can have an average molecular weight of about 5,000 to about 50,000.

In another preferred embodiment of the present invention, the polymeric composition can be used to formulate a controlled release delivery system for leuprolide mesylate. In such an embodiment, the lactate-based polymer can preferably be poly (DL-lactide-co-glycolide) containing 75% lactide in the polymer chain or higher, two hydroxyl terminal groups; can be present in about 30% to about 65% of the composition by weight; and can have an average molecular weight of about 5,000 to about 50,000.

In still another preferred embodiment of the present invention, the lactate-based biodegradable polymer of the composition has a residual lactide content of 0.2% or less and can be formulated with leuprolide mesylate. In such an embodiment, the biodegradable polymer can preferably be poly(lactide-co-glycolide) or 100/0 poly (DL-lactide) with/without carboxylic acid terminal groups; can be present in about 10% to about 65% of the composition by weight; and can have an average molecular weight of about 5,000 to about 50,000. When formulated with a pharmaceutically acceptable organic solvent, such as NMP, the formation of leuprolide-lactide conjugates through serine site is less than 5%, preferably less than 2%, more preferably less than 1%, and most preferably less than 0.5%.

In one aspect, the present invention provides stabilized injectable biodegradable polymeric compositions for forming economical, practical, and efficient controlled release delivery systems that comprise a) a bioactive substance or salt thereof; b) a pharmaceutically acceptable organic solvent; c) a lactate-based biodegradable homopolymer or copolymer. The bioactive substances or their salts thereof of the present invention are typically nucleophilic and can react with lactide monomers or lactate-based oligomers to form covalent conjugates or adducts. Preferably, the polymeric composition is injectable and can be packaged into a kit comprising a step to fill the composition into a syringe in a ready-to-use configuration. The composition in the kit is stable for a reasonable period of time, preferably at least one year, to have a suitable storage shelf-life under controlled storage conditions. The composition is preferably injected into a subject to form *in situ* an implant, from which the bioactive substance is released in a therapeutic effective amount over a desired, extended period of time.

In another preferred embodiment of the present invention, a process is provided for making an injectable composition for controlled release drug delivery comprising: combining a lactate-based polymer having a weight average molecular weight between 5,000 and 50,000 dalton, an acid number of less than 3 mgKOH/g and a residual lactide monomer in the lactate-based polymer of less than about 0.3% by weight; with a pharmaceutically acceptable organic solvent; and a bioactive substance or a salt thereof capable of reacting with lactide monomer to form a conjugate, with the proviso that no acid additive is added in making the composition. Wherein the acid additive as defined herein is not the acid existing in the lactate-based polymer or derived from the degradation of the lactate-based polymer. The acid additive is the material that needs to be added to the composition in addition to the lactate-based polymer.

In one aspect, the lactate-based polymer having an acid number of less than, preferably, 2 mgKOH/g and more preferably less than 1 mgKOH/g.

In another aspect, the lactate-based polymer having a residual lactide monomer in the lactate-based polymer of less than about 0.3% by weight, preferably less than 0.2% by weight and more preferably less than 0.1% by weight.

In further another aspect, the lactate-based polymer in which the content of oligomers having molecular weights of 1000 or less is about 2% by weight or less.

### EXAMPLES

The following examples illustrate the compositions of the present invention. The examples do not limit the invention, but are provided to teach how to make useful controlled release drug delivery compositions.

### Example 1: Leuprolide acetate in PLA polymer solution in NMP

A similar formulation as disclosed in example 6 of US Patent 6,565,874 was prepared and evaluated. A poly(DL-lactide) with a weight-average molecular weight of 14,000 (100 DL 2E, Evonik) having a residual lactide monomer content of 3.2% by weight was dissolved in N-methylpyrrolidone (NMP) to obtain a 60% solution of the polymer in NMP by weight. Then, 61.8mg of leuprolide acetate (purity 99.5%) was combined and mixed with 690.3 mg of the polymer solution to result in a liquid formulation. The formulation was stored at 37°C for one hour and then analyzed by HPLC.

The analysis was performed by adding an aliquot of about 10-20 mg of formulation to a 1.5 mL centrifuge tube. 333 uL of a mixture of 3 mL MeOH with 7 mL of ACN (Solution A) was added to the formulation aliquot and the tube was vortexed to dissolve the polymer. Then 667 µL of stability buffer (6 mL of triethylamine (TEA) and 3 mL of phosphoric acid to 1 liter of water, pH of 3.0) was added and the solution was mixed on a Lab-Line Titer plate shaker for 10 minutes at a speed setting of 10. The sample was analyzed by adding 0.5 mL of the solution to a HPLC vial so that a ~1 mg/mL of leuprolide concentration could be attained and measured. Leuprolide purity level was determined using a gradient reverse-phase UPLC or HPLC system. The leuprolide peak area was compared to the peak areas of the total number of peaks and was expressed as a percentage. The HPLC conditions were:
Instruments: Shimadzu HPLC system: Binary pump, model LC-10ADVP, Variable wavelength UV detector, model- SPD-M10AVP, Autosampler, model SIL-10ADVP

| | |
|---|---|
| Column: | YMC ODS-A C-18 4.6×250 mm, 5µ, 120Å |
| Mobile Phase: | A: 0.05% TFA in water |
| | B: 0.05% TFA in acetonitrile |

B: concentration 24% (initial) → 24% (2 min) → 30% (35 min) → 95% (37 min) → 24% (38 min) →re-equilibrate (40 min)

| | |
|---|---|
| Flow rate: | 1.0 mL/min |
| Column temp: | 40°C |
| Injection vol: | 10 µL |
| Detection: | 220 nm |
| Run time: | 40 min |

It was unexpectedly found that a significant amount of impurities were generated during 1 hour period at 37°C.

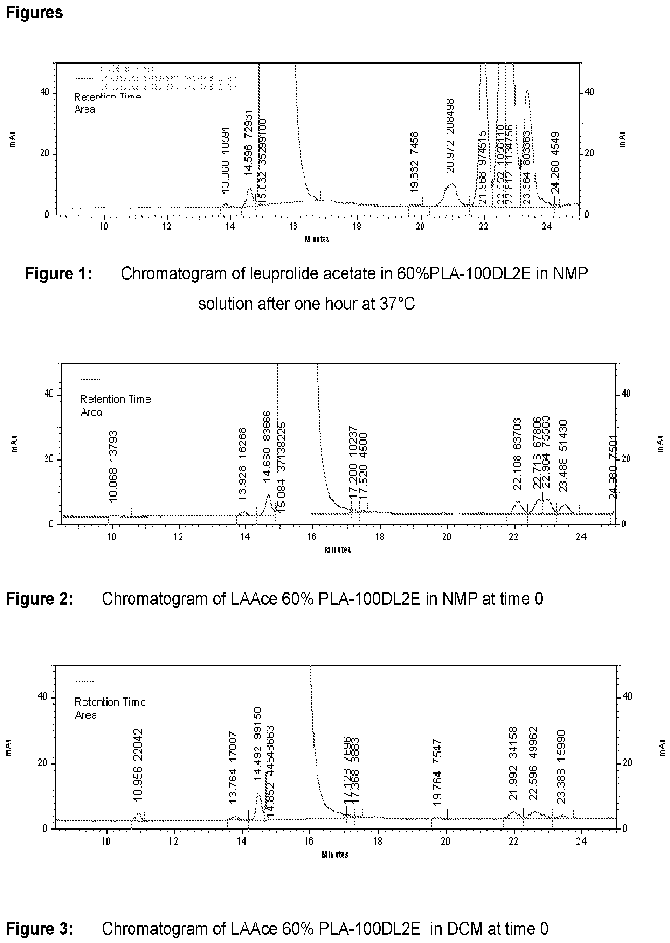

As shown in Figure 1, the retention time for leuprolide is about 15.03 min, while major leuprolide-related impurities appear at relative retention times (RRT) to leuprolide peak of approximately 1.40, 1.46, 1.50, 1.52, and 1.55. More than about 10.8% of leuprolide related impurities were generated within one hour at 37°C, as calculated by peak area. Such a level of drug related impurities would well exceed the qualification thresholds as outlined in the FDA and ICH guidelines. The significant amount of leuprolide related impurities generated from these types of formulations over such a short period of time would adversely compromise the quality of the drug product.

### Example 2: Leuprolide acetate formulated with PLA polymer in different solvents

Formulations were prepared using leuprolide acetate (LAAce) in a PLA (100 DL 2E, having a residual lactide monomer content of 3.2% by weight, Evonik) solution (60% w/w) in different solvents to test the formation of the leuprolide related impurities. The solvents tested were N-methylpyrrolidone (NMP), dichloromethane (DCM), and Dimethyl sulfoxide (DMSO). Table 1 shows the compositions of the formulations.

**Table 1: Leuprolide Acetate formulations with PLA-100DL2E in different solvents**

| **Formulation** | **Leuprolide Acetate (mg)** | **Polymer Solution (mg)** |
|---|---|---|
| **LAAce-60% PLA-100DL2E /NMP** | 61.8 | 690.3 |
| **LAAce-60% PLA-100DL2E /DCM** | 63.4 | 743.7 |
| **LAAce-60% PLA-100DL2E /DMSO** | 68.7 | 784.6 |

The formulations were mixed and stored in glass vials at 37°C. A sample was taken at time zero and analyzed by HPLC to measure the leuprolide purity. Figure 2 - Figure 4 show the initial chromatograms of the leuprolide from the formulations.

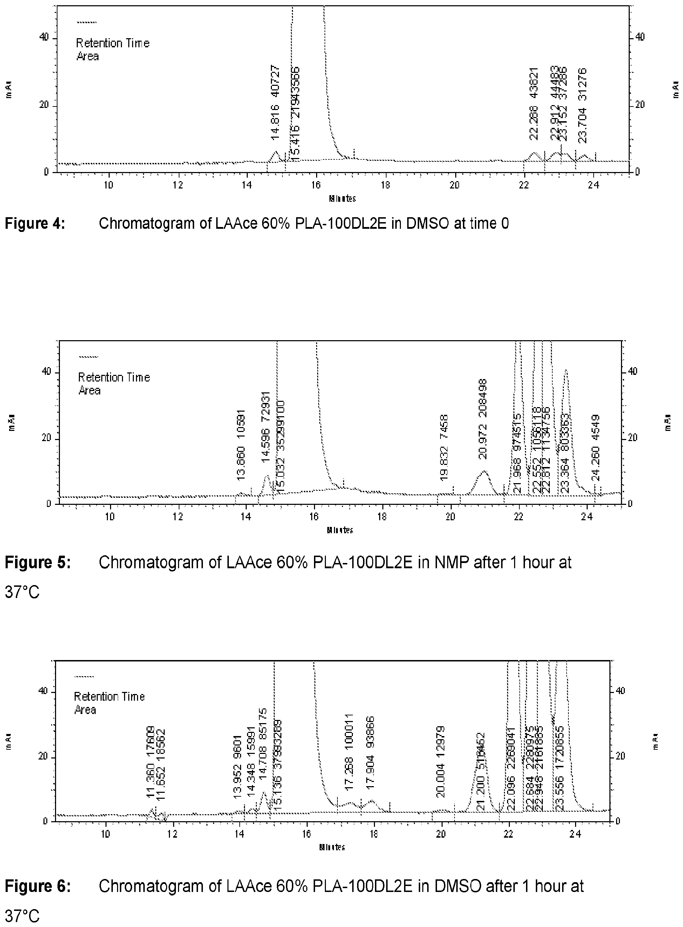

The chromatograms show that at time zero (immediately after mixing), there are already some leuprolide related impurities observed with the relative retention times (RRT) to leuprolide of 1.46, 1.49, 1.52, and 1.55. After incubation at 37°C, the formulations were again analyzed by HPLC for leuprolide.Figure 5 - Figure 7 show the chromatograms of leuprolide after 1 hour at 37°C in formulations with NMP, DMSO, and DCM, respectively.

The leuprolide related impurities observed at RRT to leuprolide peak of approximately 1.40, 1.46, 1.50, 1.52, and 1.55 are significantly more than those observed at time zero. The results show that the formation of leuprolide related impurities is much faster in the DMSO and NMP formulations than in the DCM formulation. The formation of the leuprolide related impurity in the DCM formulation did not change over the testing period. These results explain why the impurities observed in the present application are different from those disclosed in US Patent 8,343,513. In addition, DCM is not water miscible and not a pharmaceutically acceptable solvent for injection. Table 2 shows the purity of leuprolide in the formulations as determined by HPLC. The decrease in the purity of leuprolide correlates well with the increase of the leuprolide related impurities.

**Table 2: Purity of leuprolide with PLA-100DL2E in different solvents**

| **Formulation** | **Time=0** | **Time=1hr** |
|---|---|---|
| **LA-60% PLA-100DL2E /NMP** | 98.910% | 89.137% |
| **LA-60% PLA-100DL2E /DCM** | 99.425% | 99.355% |
| **LA-60% PLA-100DL2E /DMSO** | 99.025% | 80.111% |

Thus, significant impurities can develop when leuprolide is in the presence of a pharmaceutically acceptable, water miscible solvent like NMP and DMSO.

### Example 3: Arginine and Serine reaction with D,L-lactide monomers

US patent 8,343,513, Figure 16 (columns 43-44) shows the structures of impurities generated with leuprolide acetate in microspheres made from RG503H polymer in DCM solutions. All impurity structures identified have polymers reacting with the arginine group of the peptide. In the present invention, it is shown that the conjugates of lactide monomers reacting with the serine group of the peptide are the more significant impurities generated, that were not observed previously. To test the generation of leuprolide conjugates with lactide monomers, FMOC-ARG-OH or FMOC-SER-OH was dissolved in NMP. To this solution D,L-lactide monomers were added. The solution was mixed well by vortexing. 5 uL of the solution was added to an HPLC vial with 0.5 mL of acetonitrile and 0.5 mL stability buffer (0.6% TEA/0.3%H₃PO₄ in water, pH=3.0). The sample was then analyzed by HPLC. The remaining solutions were stored in a glass vial at 25°C. Samples were taken at specified time points and analyzed by UPLC. Table 3 shows the formulation compositions.

**Table 3: Serine and Arginine formulation compositions**

| **FMOC-SER-OH (mg)** | **NMP (mg)** | **D,L-lactide (mg)** |
|---|---|---|
| 95.7 | 205.0 | 100.8 |

| **FMOC-ARG-OH (mg)** | **NMP (mg)** | **D,L-lactide (mg)** |
|---|---|---|
| 97.2 | 205.3 | 100.0 |

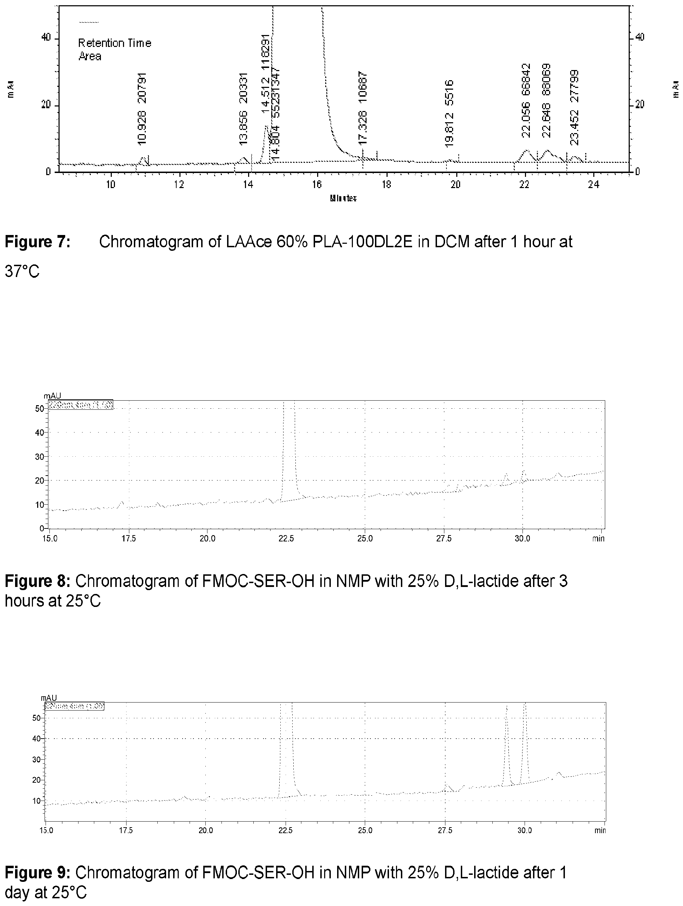

The HPLC chromatograms at 3 hours and 24 hours are shown for each formulation. Figure 8 shows the HPLC chromatogram for the FMOC-SER-OH solution after 3 hours of incubation.

Figure 8 shows there are very little impurities generated after 3 hours of incubation with the lactide monomers. The main serine peak has a retention time of 22.5 minutes. Double impurity peaks are starting to appear at 29.5 and 30.0 minutes. Figure 9 shows the chromatogram after 1 day at 25°C.

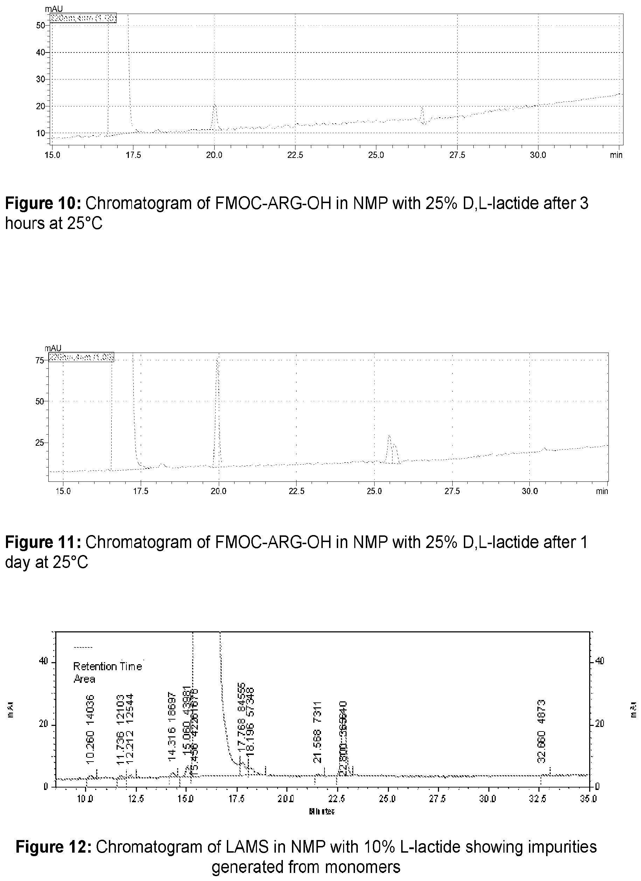

Figure 9 shows there are impurities generated from the d,l-lactide reaction with the serine at 29.5 and 30.0 minutes. The 2 peaks are from reaction of the serine with each of the monomers (D- and L-lactide). Surprisingly, this reaction generates a significant amount of impurities that were not identified in US patent 8,343,513. Figure 10 shows the chromatogram of the FMOC-ARG-OH in NMP at time 3 hours.

The arginine peak is at 16.8 minutes. While impurities are seen at 20.0 minutes, no double impurity peak is seen. Figure 11 shows the chromatogram of the same sample after 1 day at 25°C.

After 1 day, the impurity seen at 20.0 minutes has increased, as has an impurity at 25.5 min. No double peak as seen with the serine forms with arginine. Also, the overall impurity generation is still less than that seen with the serine suggesting the serine of leuprolide is more reactive with the D,L-lactide monomers in the formulation when in a pharmaceutically acceptable, water miscible solvent like NMP.

### Example 4: Leuprolide stability with PLAs of different acid numbers in NMP

US patent 8,343,513 claims a nucleophilic compound with an organic solvent and a polymer can be stabilized with additional acid. The present invention shows polymers with a higher acid number still cannot prevent the reaction of the nucleophilic compound with the residual monomers of the polymer when in a water miscible organic solvent. The polymer properties are shown in Table 4.

**Table 4: Polymer Properties**

| **Polymer** | **IV** | **Composition Lac:Gly** | **MW** | **Residual Lactide (%)** | **Acid No** (mgKOH/g) |
|---|---|---|---|---|---|
| **PLA1** | 0.22 | 100:0 | 16k | 0.16 | 1 |
| **PLA2** | 0.17 | 100:0 | 11k | 0.38 | 12 |

PLA polymers, PLA1 and PLA2 were dissolved in NMP to make a 57.5% and 60% polymer solution, respectively. Formulations were made by mixing leuprolide acetate (LAAce) (CSBio, #GF1122) into the polymer solutions. Table 5 shows the formulation compositions.

**Table 5: Leuprolide formulation composition**

| **Formulation** | **Leuprolide (mg)** | **Polymer Solution (mg)** |
|---|---|---|
| **LA-57.5%PLA1-NMP** | 85.9 | 623.9 |
| **LA-60%PLA2-NMP** | 79.3 | 589.7 |

The solutions were mixed well and stored at 37°C. At specified time points, the purity of the solution was analyzed by UPLC and polymer molecular weight was analyzed by GPC. The UPLC conditions were:
Instruments: Shimadzu UPLC system: Binary pump, model LC-30AD, Variable wavelength UV detector, model- SPD-M30A, Autosampler, model SIL-30AC

| | | |
|---|---|---|
| Column: | Acquity UPLC BEH C18 Column, 130Å, 1.7um, 3mm,x150mm | |
| Mobile Phase: | A: | Stability Buffer (6 mL of triethylamine (TEA) and 3 mL of phosphoric acid to 1 liter of water with the pH adjusted to 3.0) |
| | B: | Acetonitrile |

B: concentration 15% (initial) → 24% (40 min) → 24.9% (44 min) → 70% (46 min) → 70% (48.5 min) → 15% (49 min) →re-equilibrate (56 min)

| | |
|---|---|
| Flow rate: | 0.4 mL/min |
| Column temp: | 60°C |
| Injection vol: | 2 µL |
| Detection: | 220 nm |
| Run time: | 56 min |

Table 6 shows the relative retention times (RRT) for the peaks seen with the formulations at the specified time points.

**Table 6: RRT for Leuprolide formulations after incubation at 37°C**

| **Time** | **t=0** | | **t=24hr** | | **t=48hr** | |
|---|---|---|---|---|---|---|
| **Polymer** | **PLA1** | **PLA2** | **PLA1** | **PLA2** | **PLA1** | **PLA2** |
| Total Impurities | 0.919 | 0.992 | 3.836 | 4.069 | 4.738 | 5.253 |
| Impurity at RRT1.293 | | | **1.091** | **1.221** | **1.336** | **1.727** |
| Impurity at RRT1.307 | | | **1.149** | **1.458** | **1.356** | **1.975** |

The total impurities or lactide-leuprolide conjugates at RRT of 1.29, and 1.31 increase over time. Surprisingly, the total impurities or lactide-leuprolide conjugates increase faster for the formulation with the higher acid number.

The polymer molecular weight was analyzed by GPC. Table 7 shows the change in molecular weight over time as a percentage of the initial molecular weight.

**Table 7: Polymer molecular weight change as a percent of initial weight after incubation at 37°C**

| **Time (days)** | **PLA1** | **PLA2** |
|---|---|---|
| 0 | 100.00 | 100.00 |
| 1 | 98.21 | 95.48 |
| 2 | 96.32 | 89.71 |

Contrary to US patent 8,343,513, the polymer in the formulation with the higher acid number (PLA2) is not as stable as the polymer with lower acid number.

### Example 5: Leuprolide stability in solution with PLGA containing different amount of D,L-lactide monomers

US patent 8,343,513 claims a nucleophilic compound in a dispersed phase in an organic solvent and a polymer having acid numbers of at least 5, can be stabilized. The present invention shows the higher acid number does not prevent the formation of the impurities and lactide conjugates with leuprolide. A PLGA polymer, PLGA5050 containing different amount of residual lactide monomers was used to measure the difference in formulation stability. Table 8 shows the properties of this polymer.

**Table 8: Polymer Properties**

| **Polymer** | **IV** | **Composition Lac:Gly** | **MW** | **Residual Lactide (%)** | **Acid No** |
|---|---|---|---|---|---|
| **PLGA5050-1** | 0.37 | 51:49 | 27k | 0.02 | 5 mgKOH/g |
| **PLGA5050-2** | 0.37 | 51:49 | 27k | 0.32 | 5 mgKOH/g |

50% polymer solutions of PLGAs containing different amounts of residual D,L-lactide were prepared by dissolving the polymers in suitable amount of NMP.

Formulations were made by mixing leuprolide acetate (CSBio, #GF1122) into the polymer solutions. Table 9 shows the formulation compositions.

**Table 9: Leuprolide formulation composition**

| **Formulation** | **leuprolide (mg)** | **Polymer Solution (mg)** |
|---|---|---|
| **LA-50%PLGA5050-1/NMP** | 77.8 | 572.1 |
| **LA-50%PLGA5050-2/NMP** | 80.9 | 603.1 |

The solutions were mixed well and stored at 37°C. At specified time points, purity of the solution was analyzed by UPLC and polymer molecular weight was analyzed by GPC.

Table 10 shows the relative retention times (RRT) for the peaks seen with the formulations at the specified time points.

**Table 10: RRT for Leuprolide formulations after incubation at 37°C**

| | **t= 0 hr** | | **t= 3 hr** | | **t= 24 hr** | |
|---|---|---|---|---|---|---|
| **RRT** | **50%PLGA 5050-1** | **50% PLGA5050-2** | **50%PLGA50 50-1** | **50% PLGA5050-2** | **50%PLGA 5050-1** | **50% PLGA5050-2** |
| 0.521 | N.D. | N.D. | N.D. | N.D. | 0.076 | N.D. |
| 0.573 | 0.124 | 0.140 | 0.148 | 0.061 | 0.140 | 0.149 |
| 0.970 | N.D. | 0.048 | 0.050 | 0.047 | 0.063 | 0.051 |
| 0.978 | 0.186 | 0.188 | 0.188 | 0.188 | 0.179 | 0.167 |
| **1.000** | **99.113** | **99.053** | **98.827** | **97.912** | **93.970** | **90.969** |
| 1.040 | 0.577 | 0.571 | 0.583 | 0.585 | 1.170 | 1.155 |
| 1.090 | N.D. | N.D. | 0.137 | 0.161 | 2.197 | 1.859 |
| 1.141 | N.D. | N.D. | N.D. | N.D. | 0.136 | 0.090 |
| 1.180 | N.D. | N.D. | N.D. | N.D. | 0.046 | 0.056 |
| 1.196 | N.D. | N.D. | N.D. | N.D. | 1.129 | 0.970 |
| 1.207 | N.D. | N.D. | N.D. | N.D. | 0.090 | 0.069 |
| 1.283 | N.D. | N.D. | N.D. | N.D. | 0.057 | 0.094 |
| **1.297** | **N.D.** | **N.D.** | **0.016** | **0.479** | **0.264** | **1.964** |
| **1.312** | **N.D.** | **N.D.** | **0.018** | **0.533** | **0.313** | **2.195** |
| 1.337 | N.D. | N.D. | 0.018 | 0.533 | 0.115 | 0.159 |
| 1.364 | N.D. | N.D. | N.D. | N.D. | 0.055 | 0.052 |

| | | | | | | |
|---|---|---|---|---|---|---|
| *N.D.-none detected | | | | | | |

The lactide-leuprolide conjugates are at a RRT of 1.297, and 1.312. Again, the impurities are seen to increase over time and increase faster for the formulation containing more lactide monomers.

The polymer molecular weight was analyzed by GPC.

There is no significant difference between the MW of the two formulations at different time points.

### Example 6: L-lactide monomer impurity generation with Leuprolide

To prove if the impurities were generated from the reaction with the D,L-lactide monomers, leuprolide mesylate (LAMS) was incubated with L-lactide to see if the impurities formed showed only a single peak instead of the double peak seen previously.

Table 11 shows the composition for this solution.

**Table 11: LAMS in NMP with L-lactide composition**

| **Solution** | **Lactide (mg)** | **NMP (mg)** | **LAMS (mg)** |
|---|---|---|---|
| **LAMS** | 5.2 | 402.5 | 127.7 |

Figure 12 shows the chromatogram of LAMS in NMP with 10%L-lactide after 3 hours at 37°C.

Figure 12 shows that now the double peak seen previously is now a single peak. The double peaks mean both isomers of lactide are reacting. Figure 12 confirms it is the lactide monomer causing the impurities since the impurities are seen at the same RRT, but are only single peaks when incubated with only one of the isomers of lactide.

### Example 7: Leuprolide with different concentrations of D,L-lactide monomers

Solutions were made with leuprolide acetate (LAAc) in NMP with different amounts of D,L-lactide according to Table 12 to test the stability of leuprolide.

**Table 12: Leuprolide acetate formulation composition with lactide**

| **Solution** | **LAAc (mg)** | **NMP (mg)** | **D,L-lactide (mg)** |
|---|---|---|---|
| **1%lactide** | 156.5 | 412.5 | 5.4 |
| **0.1%lactide** | 372.5 | 986.1 | 1.3 |
| **0% lactide** | 76.6 | 207.2 | - |

The solutions were mixed well and stored at 37°C. At specified time points, a small aliquot of the solutions was added to a HPLC vial and the purity of the solution was analyzed by HPLC. Table 13 shows the purity of these formulations over time with the major impurities generated from the lactide monomers.

**Table 13: HPLC peak area percentage obtained from solution of leuprolide acetate with lactide in NMP at 37°C**

| | **Time= 0** | | | **Time= 4 hours** | | |
|---|---|---|---|---|---|---|
| **RRT** | **1%lac** | **0.1%lac** | **0%lac** | **1%lac** | **0.1%lac** | **0%lac** |
| 1.000 | 97.698 | 99.533 | 99.73 | 71.4 | 96.252 | 99.701 |
| 1.083 | 0.733 | 0.069 | N.D. | 11.605 | 1.21 | N.D. |
| 1.086 | 0.932 | 0.068 | N.D. | 15.857 | 1.965 | N.D. |

Table 13 shows the percentage of the leuprolide decreases with increasing lactide content. The impurities seen at the relative retention times (RRT) of 1.083 and 1.086 also increase with increasing lactide content. No conjugates are observed to form in the sample with no lactide monomers present during the 4 hours at 37°C.

### Example 8: Purification of lactide-based polymers

An appropriate amount of lactide-based polymer, PLA100DL2E (MW 14k, residual monomer 3.2%), was dissolved in a predetermined amount of acetone to achieve a desired concentration of lactide-based polymer solution. The concentration of the polymer can range from 5% to 50% by weight. In this example, about 25 g of the polymer was dissolved in 100 mL of acetone to form a clear solution in suitable container, such as a beaker. To this solution while stirring, about 100 mL of water was added to precipitate the polymer (Method 1) or about 40 mL of water was added to precipitate the polymer (Method 2). The supernatant was decanted off. This procedure was repeated up to 4 times. After the last decantation, the precipitated polymer was frozen and dried under vacuum for about 48 hours. The resulting polymer was characterized by GPC and the results are shown in Table 14.

**Table 14: Characteristics of non-purified and purified lactide-based polymer**

| | **Unpurified** | **Purified (Method 1)** | | **Purified (Method 2)** | |
|---|---|---|---|---|---|
| **Molecular Weight** | **PLA100DL2E** | **PLA100DL2E (x2)** | **PLA100DL2E (x4)** | **PLA100DL2E (x2)** | **PLA100DL2E (x4)** |
| >10,000 | 61.14% | 60.99% | 60.89% | 64.04% | 66.37% |
| <10,000 | 38.86% | 39.01% | 39.11% | 35.96% | 33.63% |
| <5,000 | 15.66% | 16.13% | 16.10% | 13.36% | 10.68% |
| <3,000 | 7.70% | 8.10% | 7.99% | 5.90% | 3.71% |
| <2,000 | 4.37% | 4.60% | 4.46% | 2.97% | 1.45% |
| <1,500 | 3.10% | 3.23% | 3.08% | 1.92% | 0.77% |
| <1,000 | 1.71% | 1.72% | 1.54% | 0.95% | 0.25% |
| <500 | 0.41% | 0.34% | 0.23% | 0.17% | 0.00% |
| Lactide | 3.2 | 0.26 | <0.03 | 0.30 | <0.03 |
| MW (weight-average) | 14.6k | 14.6k | 14.6k | 15.4k | 15.7k |
| Polydispersity (PD) | 2.149 | 2.159 | 2.114 | 1.959 | 1.693 |

Adding more water precipitates more of the smaller oligomers and does not result in a change in the overall polymer molecular weight. Adding less water removes more of the smaller oligomers and increases the polymer molecular weight and decreases the polydispersity.

### Example 9: Effect of leuprolide stability of polymer purification

Polymers from Example 8 were used to make polymer solutions and mixed with leuprolide to make formulations to compare the stability of the purified polymers with the unpurified polymer. 8% leuprolide acetate was mixed into a 60% polymer solution in NMP using the purified and unpurified polymers. Formulations were stored at 37°C and analyzed by HPLC to measure leuprolide stability. Table 15 shows the stability of the leuprolide at each time for each formulation.

**Table 15: Stability of leuprolide in formulations with differently purified polymers**

| | **Unpurified** | **Purified (Method 1)** | | **Purified (Method 2)** | |
|---|---|---|---|---|---|
| **Tim e (hr)** | **PLA100DL2 E** | **PLA100DL2 E (x2)** | **PLA100DL2 E (x4)** | **PLA100DL2 E (x2)** | **PLA100DL2 E (x4)** |
| 0 | 98.91 | 99.66 | 99.53 | 99.85 | 99.72 |
| 1 | 89.14 | 99.42 | 99.73 | 98.86 | 99.46 |
| 4 | | 98.29 | 99.38 | 97.46 | 99.03 |
| 24 | | 95.59 | 98.70 | 93.04 | 98.75 |

Table 15 shows purifying the polymer increases the stability of the leuprolide. The leuprolide with the unpurified polymer is already over 10% degraded after 1 hour at 37°C, while the leuprolide in the purified polymer formulations is still close to 99% after 1 hour. By 24 hours, there is some difference between the formulations with polymer purified by 2 cycles versus 4 cycles, showing there are still some monomers present, which increases the degradation rate. Thus, more purification steps result in the removal of more of the lactide monomers, which reduces the formation of leuprolide-lactide conjugates and increases the stability of the formulation. The difference in purification method is minimal in terms of the formulation stability.

### Example 10: LAMS stability with Purified PLGA polymers

Unpurified polymers were compared to polymers that were highly purified. The purification method involved dissolving the polymer in acetone and then precipitating by adding water into the acetone/polymer solution as in Example 8 method 2. This process was repeated up to three times for the PLGA polymer 8515DLG2CE-P to greatly reduce the lactide monomer content. Table 16 shows the monomer content of the polymers tested.

**Table 16: PLAs/PLGAs with residual D,L-lactide content**

| **Polymer** | **Number of Purification Cycle** | **D,L-lactide content** |
|---|---|---|
| 8515DLG2CE-P | 1 | 0.3 |
| 8515DLG2CE-P1 | 2 | 0.10 |
| 8515DLG2CE-P2 | 3 | 0.03 |
| 8515DLG2CE-P3 | 4 | <0.01 |
| 9010DLPG | 0 | 1.72 |
| 100DLPLA-1 | 1 | 0.16 |

Table 16 shows the monomer content is reduced for each subsequent purification of PLGA 8515DLG2CE-P. Formulations were made with leuprolide mesylate (LAMS) according to Table 17.

**Table 17: LAMS/PLGA formulation compositions**

| **Formulation** | **LAMS (SP-002) (mg)** | **Polymer Solution (mg)** |
|---|---|---|
| **LAMS(SP002)-60% 8515DLG2CE-P/NMP** | 51.3 | 585.5 |
| **LAMS(SP002)-60% 8515DLG2CE-P1/NMP** | 51.5 | 602.7 |
| **LAMS(SP002)-60% 8515DLG2CE-P2/NMP** | 57.6 | 663.3 |
| **LAMS(SP002)-60% 8515DLG2CE-P3/NMP** | 52.2 | 603.8 |
| **LAMS(SP002)-55% 9010DLPG /NMP** | 51.3 | 558.5 |
| **LAMS(SP002)-57.5% 100DLPLA/NMP** | 55.6 | 638.8 |

Formulations were stored in glass vials at 37°C. At specified time points, the leuprolide stability was measured and the sum of the impurities generated from the D,L-lactide monomer was tabulated as a percentage of the total AUC from the HPLC chromatogram as seen in Table 18.

**Table 18: Sum of lactide-leuprolide impurities (%) for formulations in Table 17 at 37°C**

| ***Time (days)*** | ***LAMS-55% 9010DLPG INMP*** | ***LAMS-60% 8515DLG2CE-P*/*NMP*** | ***LAMS-57.5% 100DLPLA INMP*** | ***LAMS-60% 8515DLG2CE-P1*/*NMP*** | ***LAMS-60% 8515DLG2CE-P2*/*NMP*** | ***LAMS-60% 8515DLG2CE -P3*/*NMP*** |
|---|---|---|---|---|---|---|
| 3 | 4.834 | 1.945 | 1.037 | 0.559 | 0.141 | 0.050 |
| 7 | 7.777 | 3.416 | 1.874 | 1.092 | 0.272 | 0.142 |
| 10 | 8.973 | 4.116 | 2.147 | 1.362 | 0.282 | 0.119 |
| 14 | 9.872 | 4.586 | 2.470 | 1.601 | 0.377 | 0.144 |

Table 18 shows the impurity peaks associated with the monomer directly correlate to the initial monomer concentration. Decreasing the monomer content through purification can significantly decrease the impurities in the formulation. Multiple purification steps can lower the monomer content further and, as a result, increase the formulation stability. At least two purification steps are preferred to lower the residual monomer content in order to significantly reduce the formation of lactide leuprolide conjugates.

### Example 11: Impurity formation of leuprolide in PLA formulations with different content of lactide monomers

Formulations were prepared using LAAce with PLA with different amounts of d,l-lactide, to test the reactivity of the leuprolide. Table 19 shows the composition of the formulations.

**Table 19: Formulations of LAAce in 57.5%PLAs in NMP**

| **Formulation** | **LAAce (mg)** | **Polymer Solution (mg)** | **Lactide (%)** |
|---|---|---|---|
| LAAce-57.5%PLA-0.1 /NMP | 101.1 | 741.1 | <0.1 |
| LAAce-57.5%PLA-0.2 /NMP | 99.1 | 744.6 | 0.16 |
| LAAce-57.5%PLA-0.3 /NMP | 99.7 | 735.8 | 0.3 |
| LAAce-57.5%PLA-0.5 /NMP | 100 | 738.9 | 0.5 |
| LAAce-57.5%PLA-1.0 /NMP | 99.8 | 745.4 | 1.0 |
| LAAce-57.5%PLA-3.0 /NMP | 100.5 | 745.4 | 3.0 |

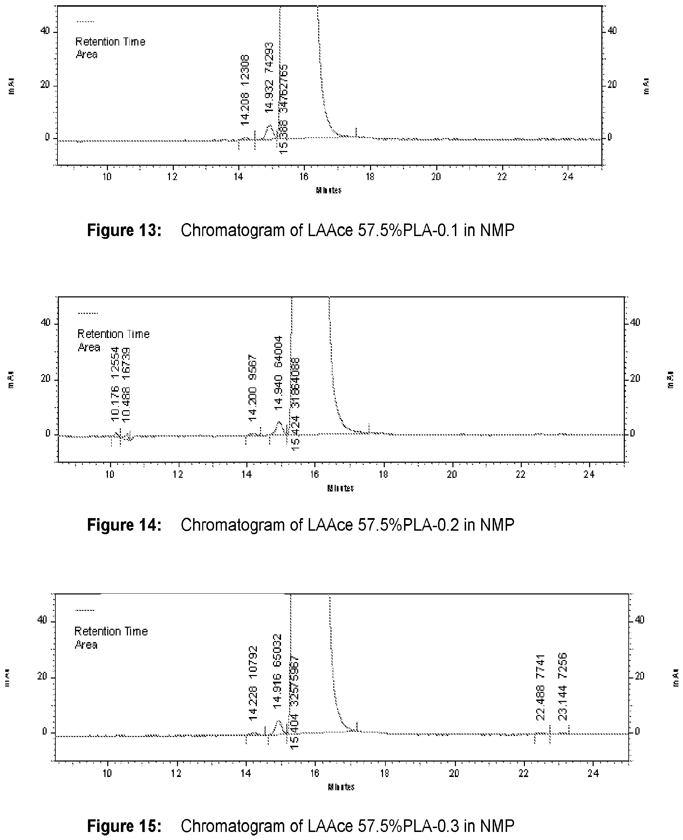

The formulations were stored in glass vials at 37°C. A sample was taken at time zero and analyzed on a HPLC to measure the leuprolide purity. Figure 13 - Figure 18 show the chromatograms of the leuprolide from the formulations initially.

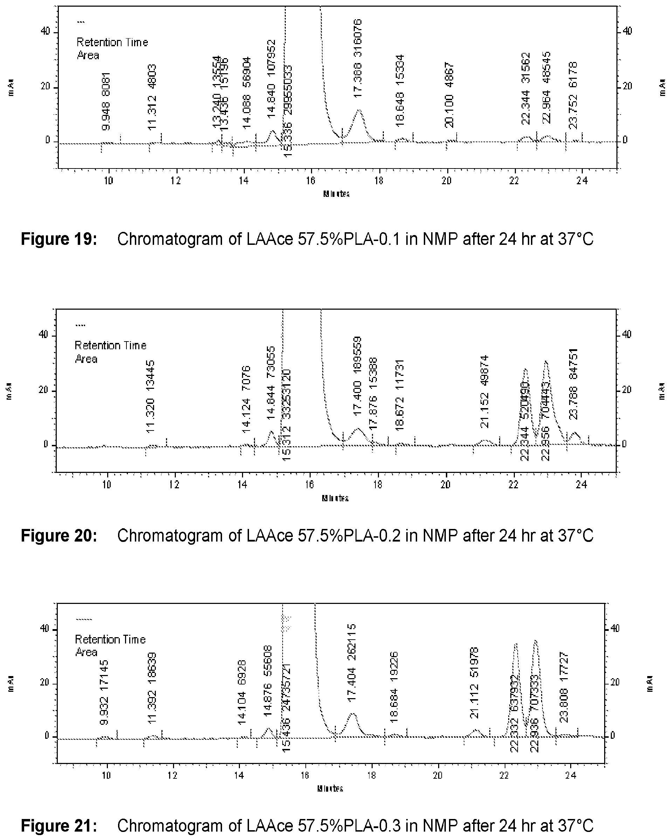

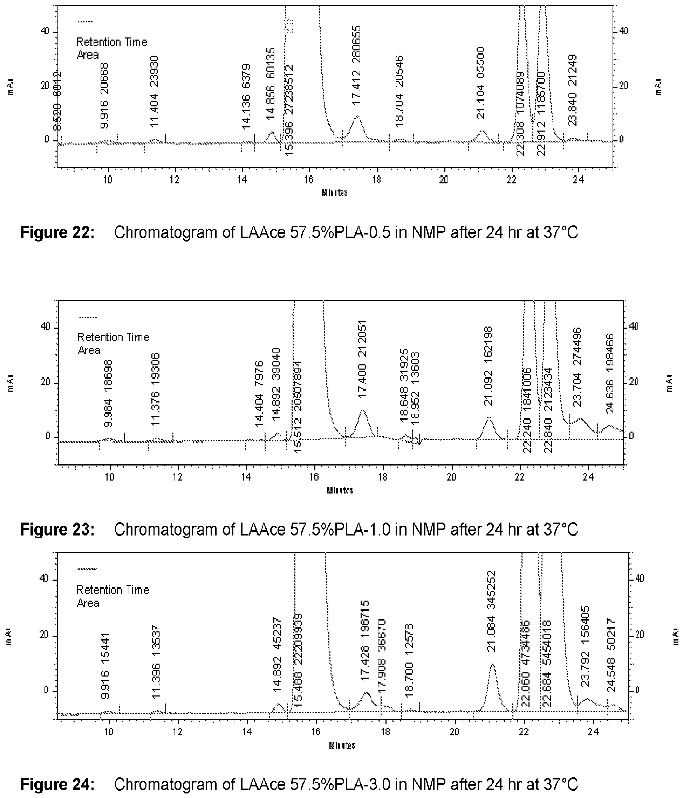

The impurities at RRT of 1.49 and 1.53 are seen to increase substantially with increasing d,l-lactide content in the formulations, even right after mixing. The samples were analyzed again after 1hr, 4hr, and 24hr. Figure 19 - Figure 24 show the 24 hr chromatograms.

Table 20 compares the leuprolide purity for the formulations at different time points in terms of the peak areas of HPLC.

**Table 20: LAAce purity at different times for formulations with different monomer concentrations**

| **Time (hr)** | **Monomer Content** | | | | | |
|---|---|---|---|---|---|---|
| | **3%** | **1%** | **0.50%** | **0.30%** | **0.16%** | **<0.1%** |
| 0 | 98.784 | 99.417 | 99.469 | 99.722 | 99.678 | 99.751 |
| 1 | 95.742 | 97.664 | 97.493 | 99.15 | 99.023 | 99.436 |
| 4 | 85.782 | 93.748 | 96.615 | 96.906 | 98.224 | 99.067 |
| 24 | 66.75 | 79.22 | 90.713 | 93.236 | 95.206 | 97.943 |

Table 21 shows the sum of the two major leuprolide lactide conjugates generated from the d,l-lactide reaction with the serine site of leuprolide.

**Table 21: Sum of two lactide-leuprolide impurities for formulations with different monomer concentrations**

| **Time (hr)** | **Monomer Content** | | | | | |
|---|---|---|---|---|---|---|
| | **3%** | **1%** | **0.50%** | **0.30%** | **0.16%** | **<0.1%** |
| 0 | 0.745 | 0.15 | 0.174 | 0.046 | - | - |
| 1 | 3.418 | 1.292 | 0.806 | 0.379 | 0.216 | - |
| 4 | 12.164 | 3.976 | 2.368 | 1.677 | 1.087 | - |
| 24 | 30.621 | 15.315 | 7.526 | 5.071 | 3.507 | 0.262 |

These tables show the impurities generated from the lactide increase over time and increase faster with the higher monomer content, showing the need for a polymer with low monomer content in the formulation.

### Example 12: Polymer Purification effect on Formulation Stability

About 25g of 8515PLGA polymer (MW 17k, residual lactide -0.15% by weight) from Durect was dissolved in about 100 mL of acetone in a glass beaker while mixing. Doubly distilled water was added 1 mL at a time to the solution. A total of 45 mL of water was added and the polymer precipitated and formed a layer on the bottom of the beaker. The solution was decanted and then re-dissolved in about 100 mL of acetone. Doubly distilled water was added again, 1 mL at a time until a total of 45 mL was added. The precipitate was decanted and centrifuged. The precipitate was washed 2 times with water and then frozen and lyophilized. The molecular weight of the purified polymer was found to have increased slightly from 17.9k to 18.3k. The content of residual lactide monomer was expected to be reduced from about 0.15% to less than 0.03% by weight.

The purified and unpurified polymers were mixed with NMP to make a 57.5% polymer solution in NMP. Leuprolide mesylate (LAMS) was added to each of the polymer solutions to make an 8% LAMS formulation with the 57.5% polymer solution. The formulations were filled into 1 mL long COC syringes from Schott with 4023/50 grey plungers from West. The syringes with formulation were then sterilized by ebeam irradiation at a dose of 27 kGy.

After irradiation, the formulations were stored at 25°C and the stability of the formulation was measured. Table 22 shows the impurities in the formulations as well as the generation of leuprolide lactide conjugate at serine site (Leup-Serine-Lac).

**Table 22: Impurity formation in sterilized LAMS formulations with purified or unpurified 8515PLGA at 25°C**

| **Time(wk)** | **Total Impurities** | | **Sum Leup-Serine-Lac conjugate** | |
|---|---|---|---|---|
| | **purified** | **unpurified** | **purified** | **unpurified** |
| 0 | 0.934 | 0.674 | 0 | 0 |
| 4 | 1.623 | 3.341 | 0.250 | 2.035 |
| 8 | 2.294 | 4.636 | 0.339 | 2.823 |
| 13 | 2.705 | 5.125 | 0.409 | 2.774 |

Table 22 shows there is a significant generation of conjugates with the unpurified polymer that is about 8-fold more than that using the purified polymer in the formulation.

The molecular weight was also measured and is seen in Table 23.

**Table 23: Molecular Weight stability in sterilized formulation with purified or unpurified 8515PLGA**

| **Time(wk)** | **Molecular Weight** | | **PDI** | | **% MW Remaining** | |
|---|---|---|---|---|---|---|
| | **purified** | **unpurified** | **purified** | **unpurified** | **purified** | **unpurified** |
| 0 | 17.0k | 16.8k | 1.84 | 1.82 | 100 | 100 |
| 4 | 16.3k | 16.2k | | | 96.1 | 96.5 |
| 8 | 16.0k | 15.9k | | | 94.3 | 94.7 |
| 13 | 15.7k | 15.3k | | | 92.2 | 91.0 |

Little difference is seen in the molecular weight between the two formulations over time. Also there is no difference in the polydispersity index between the two formulations.

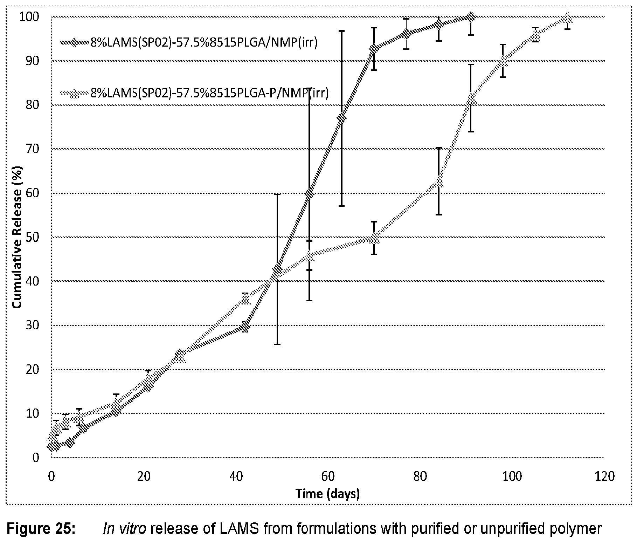

The *in vitro* release in PBS at 37°C was also measured for the two formulations and is shown in Figure 25.

Figure 25 shows the formulation with 8515PLGA-P last a few weeks longer than the formulation with the 8515PLGA. This is unexpected as the molecular weight and polydispersity of the polymers in both formulations are basically the same. The difference in the release is likely due to the removal of the small oligomers in the polymer. The removal of the small oligomers makes the polymer degradation slower in this example. This is surprising and in contrary with the teaching of prior art i.e., when "oligomer acids are incorporated into the polymer-drug solution, it can considerably reduce or eliminate the molecular weight reduction of the polymer" [See US Patent 8,343,513, Column 3, lines 44-48].

For certain therapeutics such as GnRH agonist analogs, the high initial release may be advantageous. GnRH agonist interrupt the normal pulsatile stimulation of, and thus desensitizing, the GnRH receptors, it indirectly downregulates the secretion of gonadotropins luteinizing hormone (LH) and follicle-stimulating hormone (FSH), leading to hypogonadism and thus a dramatic reduction in estradiol and testosterone levels in both sexes. Initial treatment requires higher dose of GnRH agonist to suppress testosterone levels. Once the suppression of testosterone below serum castration level (≤0.5ng/mL), only very low dose of GnRH agonist is required to maintain the castration level. Therefore, both higher initial burst release and extended delivery duration of GnRH agonists are beneficial.

## Claims

1. A process for making an injectable composition for controlled release drug delivery comprising:
combining a lactate-based polymer having a weight average molecular weight between 5,000 and 50,000 dalton, an acid number of less than 3 mgKOH/g and the content of residual lactide monomers in the lactate-based polymer of less than about 0.3% by weight; with
a pharmaceutically acceptable organic solvent; and
a bioactive substance selected from the group consisting of luteinizing hormone releasing hormone (LHRH), LHRH analogs, agonists, antagonists, and salts thereof,
with the proviso that no acid additive is added in making the composition.

2. The process of claim 1 where the lactide in the lactate-based polymer is D-lactide, D,L-lactide, L,D-lactide, L-lactide, (R,R)-Lactide, (S,S)-lactide and meso-lactide or any combination thereof.

3. The process of claim 1 where the lactate-based polymer has the content of residual lactide monomers of less than about 0.2% by weight.

4. The process of claim 1 where the lactate-based polymer has the content of residual lactide monomers of less than about 0.1% by weight.

5. The process of claim 1 where the lactate-based polymer has an acid number of less than 2 mgKOH/g.

6. The process of claim 1, wherein the lactate-based polymer is poly(lactide-co-glycolide) (PLGA) having the ratio of lactide/glycolide from 50/50 to 100/0.

7. An injectable composition for controlled release drug delivery comprising:
a. a lactate-based polymer having a weight average molecular weight between 5,000 and 50,000 dalton, an acid number of less than 3 mgKOH/g and the content of residual lactide monomers in the lactate-based polymer of less than about 0.3% by weight;
b. a pharmaceutically acceptable organic solvent; and
c. a bioactive substance selected from the group consisting of luteinizing hormone releasing hormone (LHRH), LHRH analogs, agonists, antagonists, and salts thereof,
with the proviso that no acid additive is added in producing the composition, and the composition reduces the formation of the conjugate.

8. The injectable composition of claim 7 where the lactide in the lactate-based polymer is D-lactide, D,L-lactide, L,D-lactide, L-lactide, (R,R)-Lactide, (S,S)-lactide and meso-lactide or any combination thereof.

9. The injectable composition of claim 7 where the lactate-based polymer has an acid number of less than 2 mgKOH/g.

10. The injectable composition of claim 7 where the lactate-based polymer has the content of residual lactide monomers in the lactate-based polymer of less than about 0.2% by weight.

11. The injectable composition of claim 7 where the lactate-based polymer has the content of residual lactide monomers in the lactate-based polymer of less than about 0.1% by weight.

12. The injectable composition of claim 7, wherein the lactate-based polymer is poly(lactide-co-glycolide) (PLGA) having the ratio of lactide/glycolide from 50/50 to 100/0.

13. The injectable composition of claim 7 where the bioactive substance is leuprolide or a salt thereof.

## Patentansprüche

1. Verfahren zur Herstellung einer injizierbaren Zusammensetzung zur kontrollierten Freisetzung von Arzneimitteln, umfassend:
Kombinieren eines Polymers auf Lactatbasis mit einem gewichtsmittleren Molekulargewicht zwischen 5000 und 50000 Dalton, einer Säurezahl von weniger als 3 mgKOH/g und einem Gehalt an restlichen Lactidmonomeren in dem Polymer auf Lactatbasis von weniger als etwa 0,3 Gew.-%; mit
einem pharmazeutisch akzeptablen organischen Lösungsmittel; und
einer bioaktiven Substanz, ausgewählt aus der Gruppe bestehend aus luteinisierendes Hormon freisetzendem Hormon (LHRH), LHRH-Analoga, Agonisten, Antagonisten und Salzen davon,
mit der Maßgabe, dass bei der Herstellung der Zusammensetzung kein Säureadditiv hinzugefügt wird.

2. Verfahren nach Anspruch 1, wobei das Lactid in dem Polymer auf Lactatbasis D-Lactid, D,L-Lactid, L,D-Lactid, L-Lactid, (R,R)-Lactid, (S,S)-Lactid und meso-Lactid oder eine beliebige Kombination davon ist.

3. Verfahren nach Anspruch 1, wobei das Polymer auf Lactatbasis einen Gehalt an restlichen Lactidmonomeren von weniger als etwa 0,2 Gew.-% aufweist.

4. Verfahren nach Anspruch 1, wobei das Polymer auf Lactatbasis einen Gehalt an restlichen Lactidmonomeren von weniger als etwa 0,1 Gew.-% aufweist.

5. Verfahren nach Anspruch 1, wobei das Polymer auf Lactatbasis eine Säurezahl von weniger als 2 mgKOH/g aufweist.

6. Verfahren nach Anspruch 1, wobei das Polymer auf Lactatbasis Poly(lactid-co-glycolid) (PLGA) mit einem Verhältnis von Lactid/Glycolid von 50/50 bis 100/0 ist.

7. Injizierbare Zusammensetzung zur kontrollierten Freisetzung von Arzneimitteln, umfassend:
a. ein Polymer auf Lactatbasis mit einem gewichtsmittleren Molekulargewicht zwischen 5000 und 50000 Dalton, einer Säurezahl von weniger als 3 mgKOH/g und einem Gehalt an restlichen Lactidmonomeren in dem Polymer auf Lactatbasis von weniger als etwa 0,3 Gew.-%;
b. ein pharmazeutisch akzeptables organisches Lösungsmittel; und
c. eine bioaktive Substanz, ausgewählt aus der Gruppe bestehend aus luteinisierendes Hormon freisetzendem Hormon (LHRH), LHRH-Analoga, Agonisten, Antagonisten und Salzen davon,
mit der Maßgabe, dass bei der Herstellung der Zusammensetzung kein Säureadditiv zugesetzt wird und die Zusammensetzung die Bildung des Konjugats reduziert.

8. Injizierbare Zusammensetzung nach Anspruch 7, wobei das Lactid in dem Polymer auf Lactatbasis D-Lactid, D,L-Lactid, L,D-Lactid, L-Lactid, (R,R)-Lactid, (S,S)-Lactid und meso-Lactid oder eine Kombination davon ist.

9. Injizierbare Zusammensetzung nach Anspruch 7, wobei das Polymer auf Lactatbasis eine Säurezahl von weniger als 2 mgKOH/g aufweist.

10. Injizierbare Zusammensetzung nach Anspruch 7, wobei das Polymer auf Lactatbasis einen Gehalt an restlichen Lactidmonomeren in dem Polymer auf Lactatbasis von weniger als etwa 0,2 Gew.-% aufweist.

11. Injizierbare Zusammensetzung nach Anspruch 7, wobei das Polymer auf Lactatbasis einen Gehalt an restlichen Lactidmonomeren in dem Polymer auf Lactatbasis von weniger als etwa 0,1 Gew.-% aufweist.

12. Injizierbare Zusammensetzung nach Anspruch 7, wobei das Polymer auf Lactatbasis Poly(lactid-co-glycolid) (PLGA) mit einem Verhältnis von Lactid/Glycolid von 50/50 bis 100/0 ist.

13. Injizierbare Zusammensetzung nach Anspruch 7, wobei die bioaktive Substanz Leuprolid oder ein Salz davon ist.

## Revendications

1. Procédé de fabrication d'une composition injectable pour l'administration de médicaments à libération contrôlée, comprenant :
combiner un polymère à base de lactate ayant une masse moléculaire moyenne en poids comprise entre 5 000 et 50 000 daltons, un indice d'acidité inférieur à 3 mgKOH/g et une teneur en monomères de lactide résiduels dans le polymère à base de lactate inférieure à environ 0,3 % en poids ; avec
un solvant organique pharmaceutiquement acceptable ; et
une substance bioactive choisie dans le groupe constitué par l'hormone de libération de l'hormone lutéinisante (LHRH), les analogues de LHRH, les agonistes, les antagonistes et leurs sels,
à condition qu'aucun additif acide ne soit ajouté lors de la fabrication de la composition.

2. Procédé de la revendication 1 dans lequel le lactide du polymère à base de lactate est le D-lactide, le D,L-lactide, le L,D-lactide, le L-lactide, le (R,R)-lactide, le (S,S)-lactide et le méso-lactide ou toute combinaison de ceux-ci.

3. Procédé de la revendication 1 dans lequel le polymère à base de lactate a une teneur en monomères de lactide résiduels inférieure à environ 0,2 % en poids.

4. Procédé de la revendication 1 dans lequel le polymère à base de lactate a une teneur en monomères de lactide résiduels inférieure à environ 0,1 % en poids.

5. Procédé de la revendication 1 dans lequel le polymère à base de lactate a un indice d'acidité inférieur à 2 mgKOH/g.

6. Procédé de la revendication 1, dans lequel le polymère à base de lactate est un poly(lactide-co-glycolide) (PLGA) ayant le rapport lactide/glycolide compris entre 50/50 et 100/0.

7. Composition injectable pour l'administration de médicaments à libération contrôlée comprenant :
a. un polymère à base de lactate ayant une masse moléculaire moyenne en poids comprise entre 5 000 et 50 000 daltons, un indice d'acidité inférieur à 3 mgKOH/g et une teneur en monomères de lactide résiduels dans le polymère à base de lactate inférieure à environ 0,3 % en poids ;
b. un solvant organique pharmaceutiquement acceptable ; et
c. une substance bioactive choisie dans le groupe constitué par l'hormone de libération de l'hormone lutéinisante (LHRH), les analogues de LHRH, les agonistes, les antagonistes et leurs sels,
à condition qu'aucun additif acide ne soit ajouté lors de la fabrication de la composition, et que la composition réduise la formation du conjugué.

8. Composition injectable de la revendication 7 dans laquelle le lactide du polymère à base de lactate est le D-lactide, le D,L-lactide, le L,D-lactide, le L-lactide, le (R,R)-Lactide, le (S,S)-lactide et le méso-lactide ou toute combinaison de ceux-ci.

9. Composition injectable de la revendication 7 dans laquelle le polymère à base de lactate a un indice d'acidité inférieur à 2 mgKOH/g.

10. Composition injectable de la revendication 7 dans laquelle le polymère à base de lactate a une teneur en monomères de lactide résiduels dans le polymère à base de lactate inférieure à environ 0,2 % en poids.

11. Composition injectable de la revendication 7 dans laquelle le polymère à base de lactate a une teneur en monomères de lactide résiduels dans le polymère à base de lactate inférieure à environ 0,1 % en poids.

12. Composition injectable de la revendication 7, dans laquelle le polymère à base de lactate est un poly(lactide-co-glycolide) (PLGA) ayant le rapport lactide/glycolide compris entre 50/50 et 100/0.

13. Composition injectable de la revendication 7 dans laquelle la substance bioactive est le leuprolide ou un de ses sels.
